# EUROPEAN PATENT APPLICATION

(11) **EP 4 643 876 A1**
(43) Date of publication of application: **05.11.2025**
(21) Application number: 23910575.2
(22) Date of filing: 26.12.2023
(51) Int. Cl.: A61K 39/395, A61K 9/08, A61K 47/18, A61K 47/26, C07K 16/28, A61P 35/00

(54) **PHARMACEUTICAL PREPARATION COMPRISING ANTI-TIGIT ANTIBODY**

(30) Priority: 27.12.2022 CN 202211713441
(71) Applicant: Shanghai Henlius Biotech, Inc., Shanghai 201210 (CN); Shanghai Henlius Biopharmaceutical Co., Ltd., Shanghai 200233 (CN); Shanghai Henlius Biologics Co., Ltd., Shanghai 201616 (CN)
(72) Inventor: PU, Tianning, Shanghai 201210 (CN); FANG, Yuan, Shanghai 201210 (CN); HAN, Dongmei, Shanghai 201210 (CN)
(74) Representative: Plougmann Vingtoft a/s
(86) International application number: PCT/CN2023/141838
(87) International publication number: WO 2024/140651

(57) **Abstract**

A pharmaceutical preparation comprising an anti-TIGIT antibody, comprising an anti-TIGIT antibody, a stabilizer, a buffer solution, and a surfactant.

## Description

### CROSS-REFERENCE TO RELATED APPLICATION

The present application claims priority to the Chinese Patent Application No. CN202211713441.7 filed on Dec. 27, 2022, the content of which is incorporated herein by reference in its entirety.

### TECHNICAL FIELD

The present disclosure relates to a pharmaceutical formulation comprising an anti-TIGIT antibody and use of the same.

### BACKGROUND

T cell immunoreceptor with Ig and ITIM domains (TIGIT) is an immune checkpoint receptor expressed by immune cells (e.g., activated T cells and natural killer cells (NK cells)) and mediates immune suppression. The ligand of TIGIT (PVR, CD155) has been identified in dendritic cells (DCs), macrophages, and many human cancer cells. Studies have shown that the binding of the ligand to TIGIT can downregulate T cell activation and cytokine secretion. Inhibition of the TIGIT/PVR interaction can mediate potent anti-tumor activity of immune cells. Given the critical role of TIGIT in immune checkpoint regulation, therapeutic molecules and methods for modulating TIGIT-mediated immune cell suppression have been developed in the art for use in immunotherapy and cancer treatment, providing patients with more drug options.

Protein (monoclonal antibody, etc.) pharmaceutical formulations are suitable for parenteral administration, including administration routes such as intravenous, intramuscular, intraperitoneal, or subcutaneous injection. While protein liquid formulations are convenient to use, proteins in such liquid formulations are at risk of aggregation and degradation, which may lead to reduced stability. In conventional pharmaceutical formulations, the contained proteins, particularly monoclonal antibodies, are always very hard to maintain good physical, chemical, and biological stability during storage, especially under non-refrigerated conditions. Therefore, protein pharmaceutical formulations require stringent storage conditions and usually need to be stored at low temperatures. Additionally, protective agents are added to protein (antibody) pharmaceutical formulations to enhance protein stability. Protective agents that have been reported include surfactants, sugars and polyols, salts, amino acids/amino acid salts, and some macromolecular compounds. However, due to the structural and functional diversity of antibody drugs, there is still a lack of universal protein formulations that meet the demands of the pharmaceutical industry. Thus, there is an urgent need in the art for pharmaceutical formulations that stabilize antibody drugs, particularly those comprising an anti-TIGIT antibody, to maintain the stability of the anti-TIGIT antibody. Through a large number of experiments, the present application has discovered an excellent pharmaceutical formulation comprising an anti-TIGIT antibody, thereby fulfilling this need.

### SUMMARY

The present disclosure provides a pharmaceutical formulation comprising an anti-TIGIT antibody, which is capable of maintaining the physical and chemical stability of the anti-TIGIT antibody under long-term storage and various environmental conditions.

In one aspect, the present disclosure provides a pharmaceutical formulation comprising an anti-TIGIT antibody, which comprises an anti-TIGIT antibody, a buffer, a stabilizer, a surfactant, and the like.

In some specific embodiments of the present disclosure, in the pharmaceutical formulation comprising the anti-TIGIT antibody provided by the present disclosure, the comprised anti-TIGIT antibody is a single-domain antibody, and a heavy chain variable region (VHH) of the anti-TIGIT antibody comprises or consists of an amino acid sequence set forth in SEQ ID NO: 1. In some specific embodiments of the present disclosure, the anti-TIGIT antibody of the pharmaceutical formulation has a concentration in the range of 15-25 mg/mL, preferably a concentration of 20 mg/mL. In some specific embodiments of the present disclosure, a heavy chain of the anti-TIGIT antibody involved in the pharmaceutical formulation comprises an amino acid sequence set forth in SEQ ID NO: 2.

In some specific embodiments of the present disclosure, in the pharmaceutical formulation comprising the anti-TIGIT antibody provided by the present disclosure, the buffer is an acetate buffer or a histidine buffer, the stabilizer is sucrose, sorbitol, or proline, and the surfactant is polysorbate 20 or polysorbate 80.

More specifically, the present disclosure provides a pharmaceutical formulation, which comprises the following components:
(i) an anti-TIGIT antibody, wherein the anti-TIGIT antibody is a single-domain antibody, and a heavy chain variable region of the anti-TIGIT antibody comprises or consists of an amino acid sequence set forth in SEQ ID NO: 1;
(ii) an acetate buffer or a histidine buffer;
(iii) sucrose, sorbitol, or proline; and
(iv) polysorbate 20 or polysorbate 80;
wherein the pharmaceutical formulation has a pH value of 5.1 to 5.7.

In some specific embodiments of the present disclosure, the pharmaceutical formulation has a pH value of 5.1, 5.3, 5.4, or 5.7, preferably 5.3 or 5.4, and most preferably 5.4.

In some specific embodiments of the present disclosure, the buffer has a concentration of 5-15 mM, preferably 10 mM or 15 mM, and most preferably 10 mM.

In some specific embodiments of the present disclosure, the component (iii) is sucrose.

In some specific embodiments of the present disclosure, the component (iii) has a concentration of 2% (w/v)-6% (w/v), preferably 2% (w/v), 4% (w/v), or 6% (w/v), and more preferably 2% (w/v).

In some specific embodiments of the present disclosure, the component (iv) is polysorbate 80.

In some specific embodiments of the present disclosure, the component (iv) has a concentration of 0.01% (w/v)-0.05% (w/v), preferably 0.03% (w/v) or 0.05% (w/v), and more preferably 0.03% (w/v).

In some specific embodiments of the present disclosure, the anti-TIGIT antibody has a concentration of 15-25 mg/mL, preferably 20 mg/mL.

In some specific embodiments of the present disclosure, a full-length heavy chain of the anti-TIGIT antibody comprises or consists of an amino acid sequence set forth in SEQ ID NO: 2.

In some preferred embodiments of the present disclosure, the present disclosure provides a pharmaceutical formulation comprising an anti-TIGIT antibody, which comprises:
(i) an anti-TIGIT antibody having a concentration of 15-25 mg/mL;
(ii) a histidine buffer having a concentration of 5-15 mM;
(iii) sucrose having a concentration of 2%-6% (w/v); and
(iv) polysorbate 80 having a concentration of 0.01%-0.05% (w/v);
wherein the pharmaceutical formulation has a pH value of 5.1 to 5.7.

The present disclosure provides a pharmaceutical formulation comprising an anti-TIGIT antibody, which comprises
(i) an anti-TIGIT antibody having a concentration of 20 mg/mL;
(ii) a histidine buffer having a concentration of 10 mM;
(iii) sucrose having a concentration of 2% (w/v); and
(iv) polysorbate 80 having a concentration of 0.03% (w/v);
wherein the pharmaceutical formulation has a pH value of 5.4.

In another aspect, the present disclosure relates to a method for reducing tumor burden in a patient or treating a tumor in a patient, which comprises administering to the patient an effective dose of the pharmaceutical formulation described above. In the method, the pharmaceutical formulation described above is administered in combination with an additional agent; preferably, the pharmaceutical formulation is administered in combination with an anti-PD-1 antibody and/or an anti-PD-L1 antibody.

The present disclosure also relates to use of the pharmaceutical formulation described above in manufacture of a medicament for treating a cancer. In the use, the pharmaceutical formulation described above is administered in combination with an additional agent; preferably, the pharmaceutical formulation is administered in combination with an anti-PD-1 antibody and/or an anti-PD-L1 antibody.

In the method or use, the cancer or the tumor is a solid tumor or a hematological tumor.

In the method or use, the cancer or the tumor is selected from the group consisting of mesothelioma, lung cancer, pancreatic cancer, ovarian cancer, breast cancer, colon cancer, pleural tumor, glioblastoma, esophageal cancer, synovial sarcoma, thymic carcinoma, cervical cancer, endometrial cancer, gastric tumor, melanoma, cholangiocarcinoma, head and neck cancer, lymphoma, hematological cancer, and a combination thereof.

The present disclosure also relates to a drug delivery device comprising the pharmaceutical formulation.

The high-temperature accelerated testing, as well as the repeated freeze-thaw and long-term frozen storage stability studies, demonstrate that the anti-TIGIT antibody protein in the pharmaceutical formulation provided by the present disclosure maintains good stability.

Surprisingly, the pharmaceutical formulation comprising the anti-TIGIT antibody unexpectedly exhibits advantages including minimal aggregation, minimal particles, lowest turbidity, least acidic variants, minimal pH drift, highest thermostability, highest high-temperature accelerated stability, highest freeze-thaw stability, and highest long-term frozen storage stability.

### BRIEF DESCRIPTION OF THE DRAWINGS

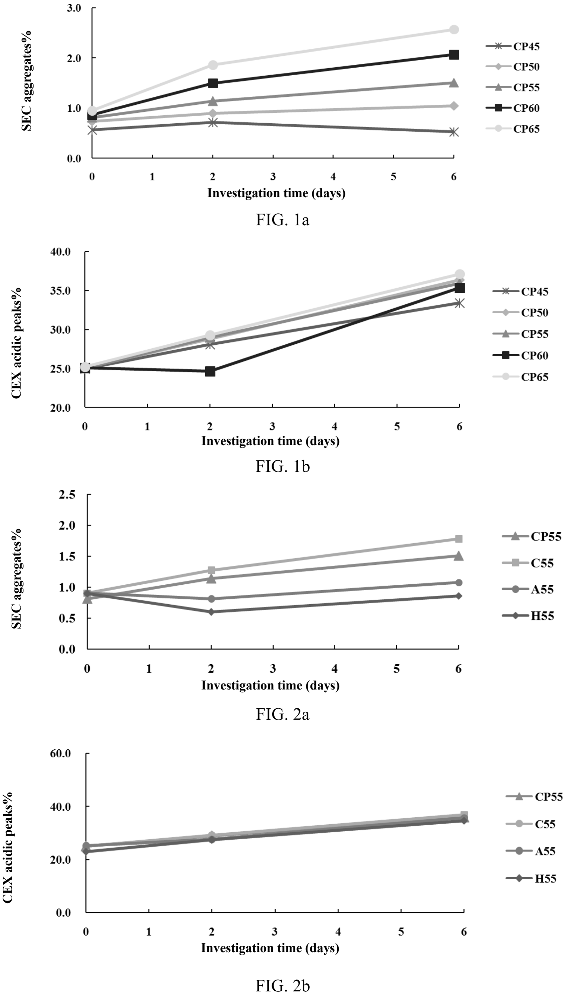

FIG. 1a shows the results of changes in SEC aggregate% over time for the pharmaceutical formulations comprising the anti-TIGIT antibody in Example 1 during the high-temperature accelerated testing; FIG. 1b shows the results of changes in CEX acidic peak% over time for the pharmaceutical formulations comprising the anti-TIGIT antibody in Example 1 during the high-temperature accelerated testing; where CP45 represents the pharmaceutical formulation having a pH value of 4.5, CP50 represents the pharmaceutical formulation having a pH value of 5.0, CP55 represents the pharmaceutical formulation having a pH value of 5.5, CP60 represents the pharmaceutical formulation having a pH value of 5.0, and CP65 represents the pharmaceutical formulation having a pH value of 6.5.
FIG. 2a shows the results of changes in SEC aggregate% over time for the pharmaceutical formulations comprising the anti-TIGIT antibody in Example 2 during the high-temperature accelerated testing; FIG. 2b shows the results of changes in CEX acidic peak% over time for the pharmaceutical formulations comprising the anti-TIGIT antibody in Example 2 during the high-temperature accelerated testing; where CP55 represents the pharmaceutical formulation comprising a citric acid-sodium dihydrogen phosphate buffer, C55 represents the pharmaceutical formulation comprising a citrate buffer, A55 represents the pharmaceutical formulation comprising an acetate buffer, and H55 represents the pharmaceutical formulation comprising a histidine buffer.

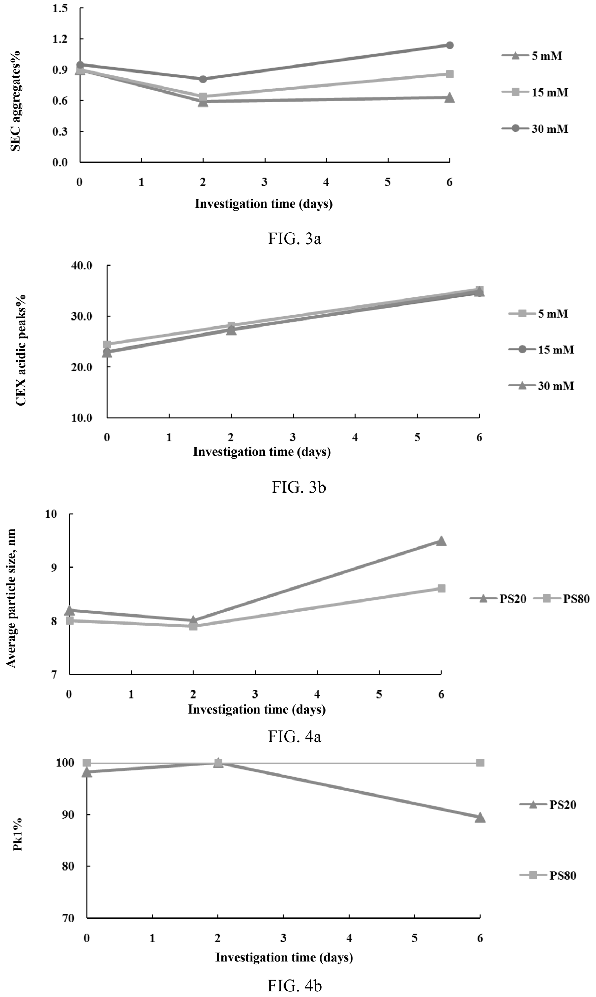

FIG. 3a shows the results of changes in SEC aggregate% over time for the pharmaceutical formulations comprising the anti-TIGIT antibody in Example 3 during the high-temperature accelerated testing; FIG. 3b shows the results of changes in CEX acidic peak% over time for the pharmaceutical formulations comprising the anti-TIGIT antibody in Example 3 during the high-temperature accelerated testing; where 5 mM represents the pharmaceutical formulation having a buffer concentration of 5 mM, 15 mM represents the pharmaceutical formulation having a buffer concentration of 15 mM, and 30 mM represents the pharmaceutical formulation having a buffer concentration of 30 mM.
FIGs. 4a and 4b show the results of changes in the average protein particle size and Pk1% over time, respectively, for the pharmaceutical formulations comprising the anti-TIGIT antibody in Example 4 during the high-temperature accelerated testing, where PS20 represents the pharmaceutical formulation comprising polysorbate 20, and PS80 represents the pharmaceutical formulation comprising polysorbate 80.

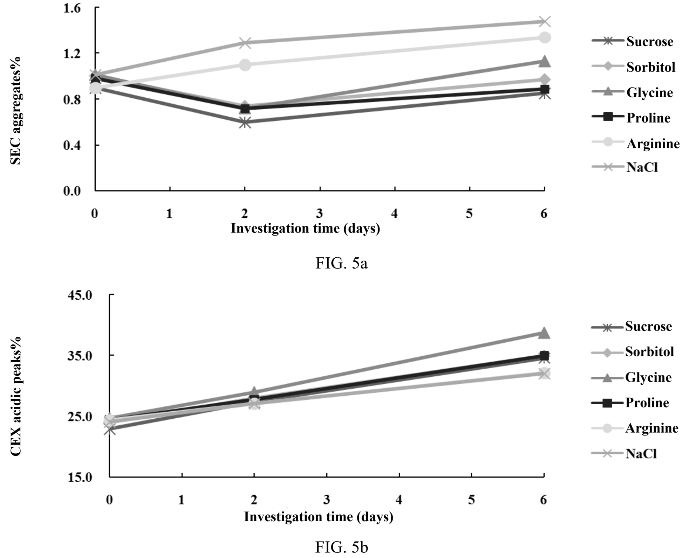

FIG. 5a shows the results of changes in SEC aggregate% over time for the pharmaceutical formulations comprising the anti-TIGIT antibody in Example 5 during the high-temperature accelerated testing; FIG. 5b shows the results of changes in CEX acidic peak% over time for the pharmaceutical formulations comprising the anti-TIGIT antibody in Example 5 during the high-temperature accelerated testing.

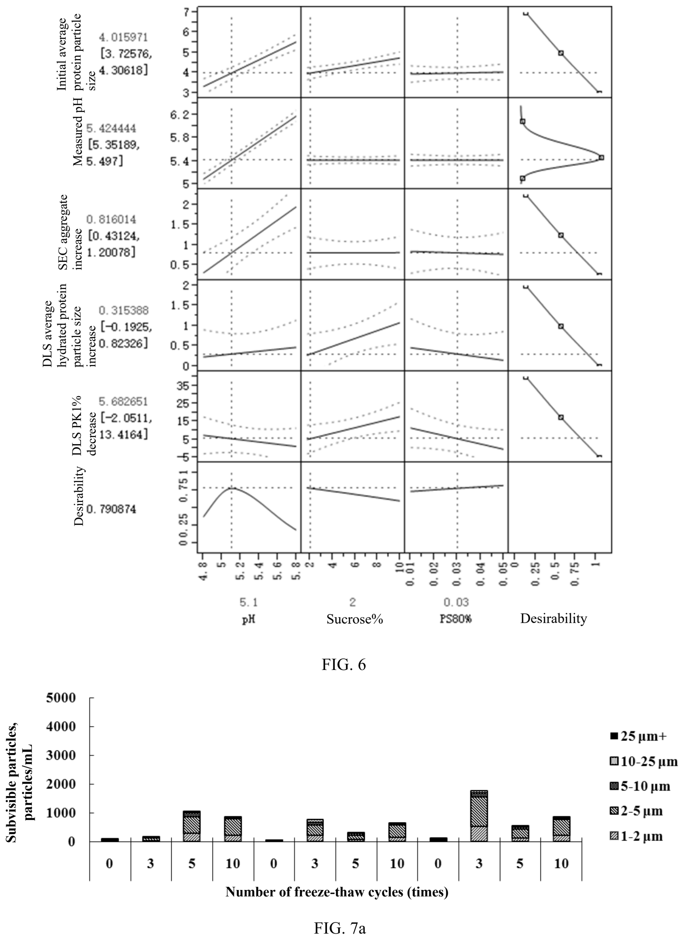

FIG. 6 shows the JMP software desirability profiler model diagram for the screening of the pH value, stabilizer concentration, and surfactant concentration of the pharmaceutical formulations comprising the anti-TIGIT antibody in Example 6.

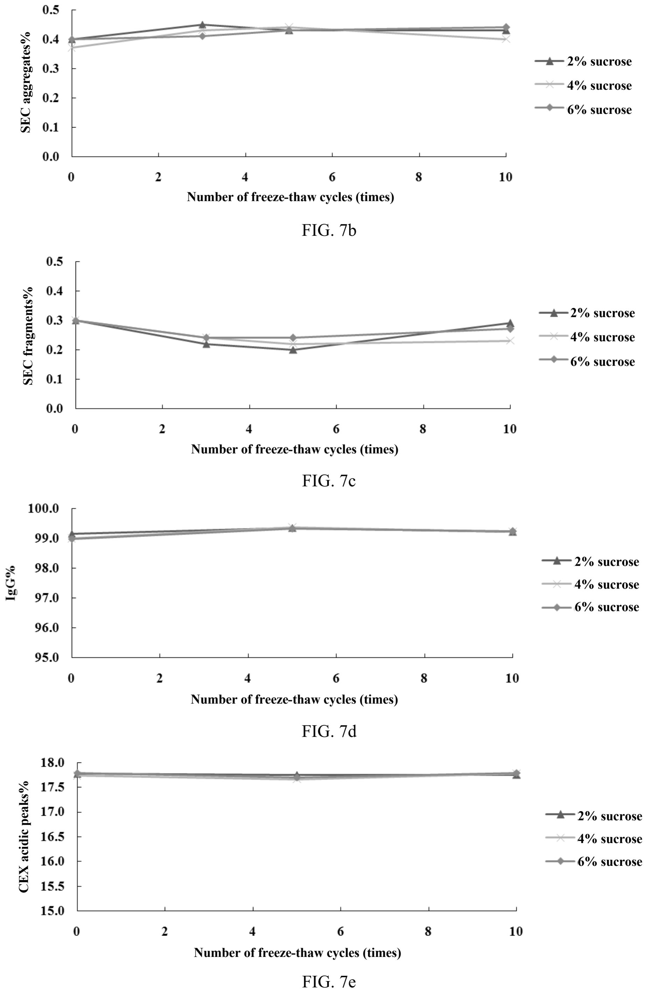

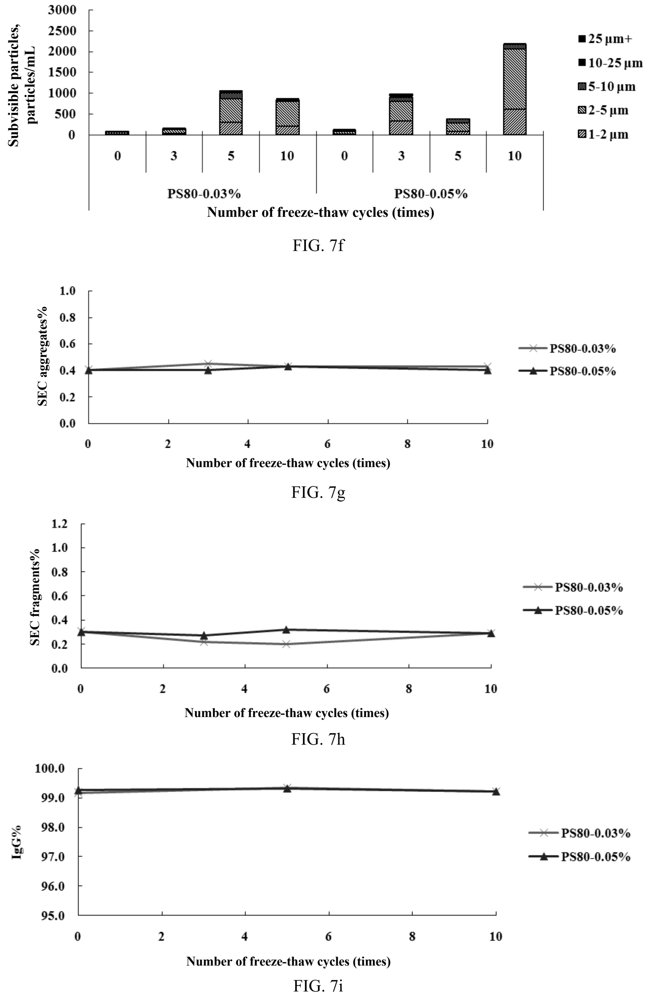

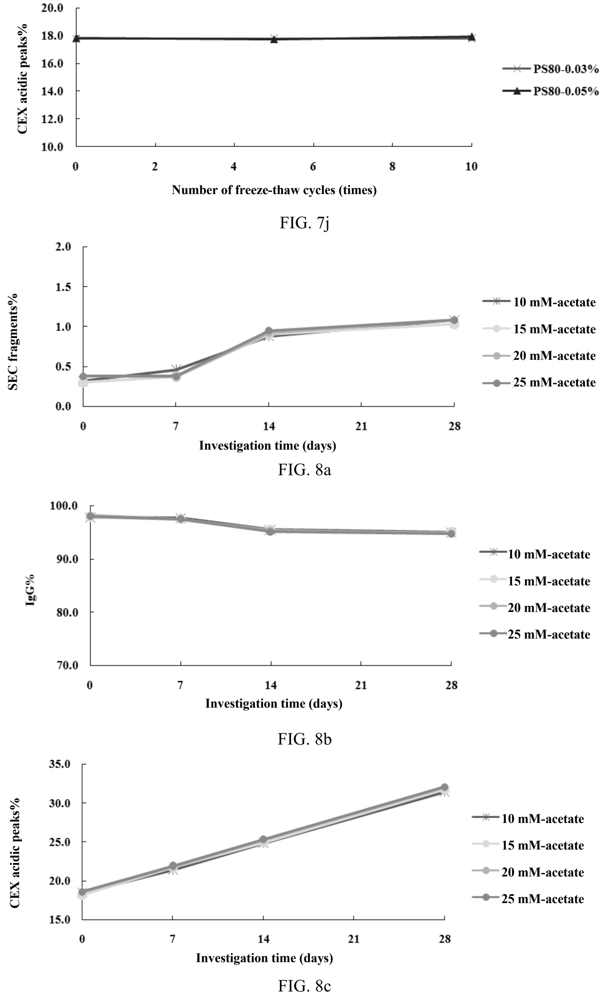

FIGs. 7a, 7b, 7c, 7d, and 7e show the results of changes in the subvisible particle (SVP) levels, SEC aggregates, SEC fragments, IgG%, and CEX acidic peaks in the formulations over time, respectively, for the pharmaceutical formulations comprising the anti-TIGIT antibody in Example 7 after repeated freeze-thaw cycles; FIGs. 7f, 7g, 7h, 7i, and 7j show the results of changes in the subvisible particle levels, SEC aggregates, SEC fragments, IgG%, and CEX acidic peaks in the formulations over time, respectively, for the other pharmaceutical formulations comprising the anti-TIGIT antibody in Example 7 after repeated freeze-thaw cycles.

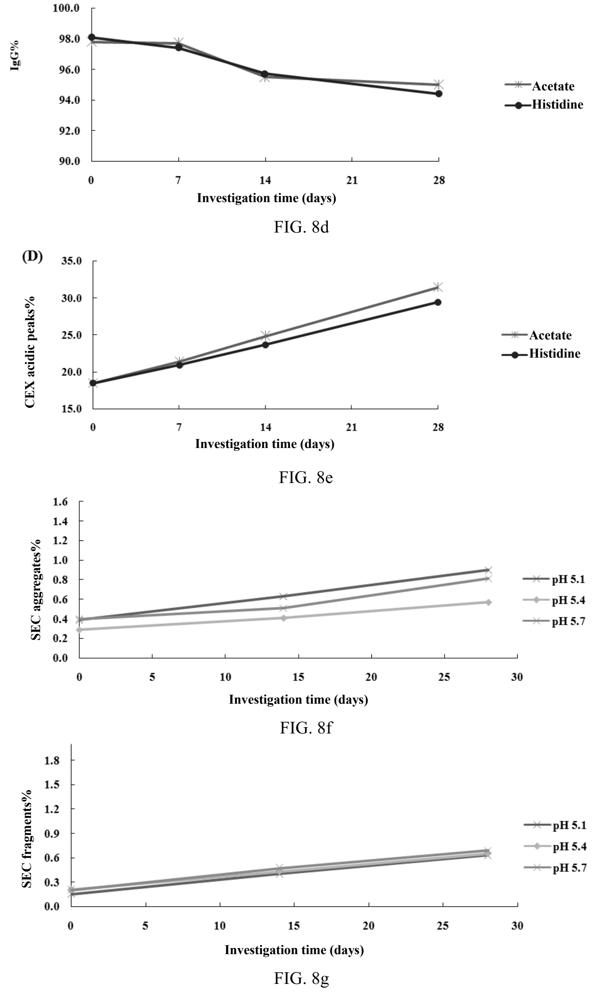

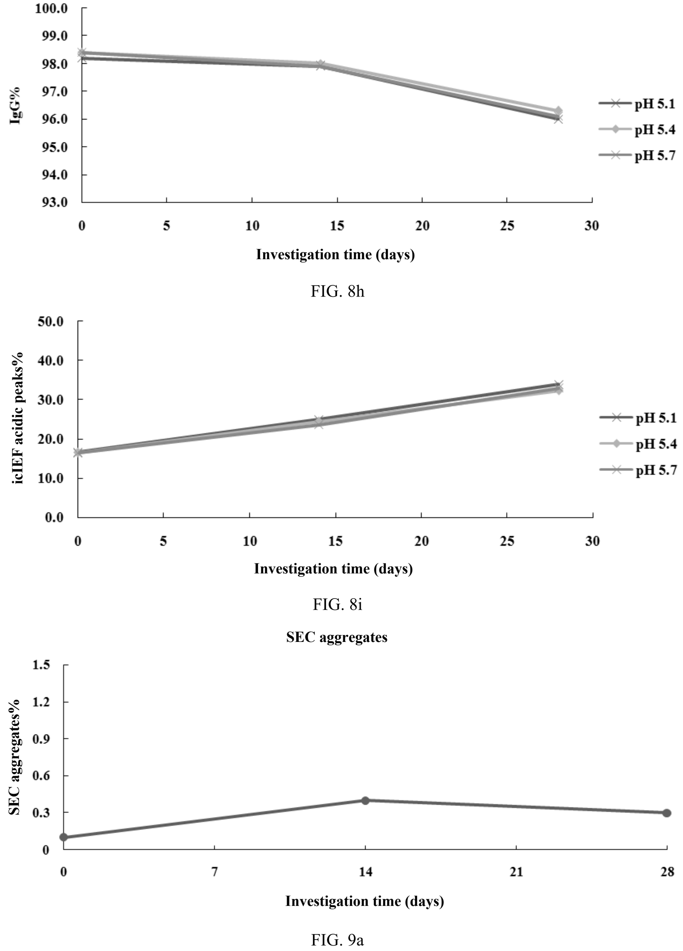

FIGs. 8a, 8b, and 8c show the results of changes in SEC fragments, IgG%, and CEX acidic peaks over time, respectively, for the pharmaceutical formulations comprising the anti-TIGIT antibody in Example 8 during the high-temperature accelerated testing; FIGs. 8d and 8e show the results of changes in CEX acidic peaks and SEC aggregates over time, respectively, for the pharmaceutical formulations comprising the anti-TIGIT antibody in Example 8 during the high-temperature accelerated testing; FIGs. 8f, 8g, 8h, and 8i show the results of changes in SEC aggregates, SEC fragments, IgG%, and icIEF acidic peaks over time, respectively, for the pharmaceutical formulations comprising the anti-TIGIT antibody in Example 8 during the high-temperature accelerated testing.

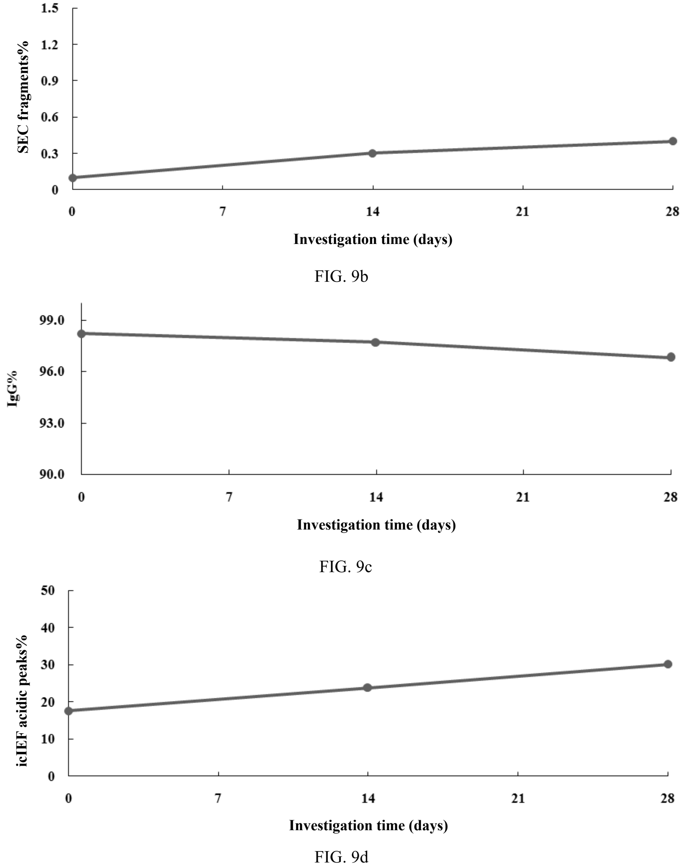

FIGs. 9a, 9b, 9c, and 9d show the results of changes in SEC aggregates, SEC fragments, IgG%, and icIEF acidic peaks over time, respectively, for the preferred pharmaceutical formulation comprising the anti-TIGIT antibody of the present disclosure during the high-temperature accelerated testing.

### DETAILED DESCRIPTION

The present disclosure will be further described in detail with reference to the following embodiments, which should not be construed as limiting the present disclosure.

### Definitions

As used herein, "pharmaceutical formulation" or "formulation" refers to a sterile composition that is suitable for parenteral administration (including but not limited to intravenous, intramuscular, subcutaneous, nebulized, and pulmonary administration) and comprises a pharmaceutically active drug; for example, the formulation comprises a biologically active protein, as well as a pharmaceutically acceptable excipient, a diluent, and an additional additive. For example, the pharmaceutical formulation may comprise a liquid (e.g., an aqueous solution that can be directly administered) and a lyophilized powder.

As used herein, "antibody" includes an intact antibody and any other antigen-binding fragment; for example, the antibody includes a monoclonal antibody (including a human or murine antibody, a humanized antibody, or a chimeric antibody), a polyclonal antibody, a multispecific antibody, an antibody-binding fragment (including but not limited to Fv, Fab, Fab', F(ab')₂, a diabody, a linear antibody, and a single-chain antibody molecule (e.g., scFv)), and a multispecific antibody formed from antibody fragments.

As used herein, terms such as "anti-TIGIT antibody" or "anti-TIGIT antibody protein" refer to an antibody that is capable of binding to TIGIT with sufficient affinity such that the antibody can be used as a diagnostic and/or therapeutic agent targeting TIGIT. The antibody specifically binding to TIGIT involved in the specific embodiments of the present disclosure is a single-domain antibody, and the heavy chain variable region of the antibody comprises or consists of an amino acid sequence set forth in SEQ ID NO: 1. In some specific embodiments of the present disclosure, the full-length heavy chain of the anti-TIGIT antibody comprises or consists of an amino acid sequence set forth in SEQ ID NO: 2. Herein, the single-domain antibody, also known as a nanobody, refers to a heavy chain single-domain antibody comprising only a VHH region or fragment, wherein the VHH may comprise a heavy chain variable region of a camelid heavy chain antibody. The anti-TIGIT antibody involved in the present disclosure is described in patent application WO2021139777A1, which is incorporated herein by reference in its entirety.

As used herein, "stabilizer" is a reagent capable of preventing or reducing chemical and/or physical instability of proteins. The stabilizer includes saccharides, alcohols, acids, salts, and the like. In an exemplary embodiment of the present disclosure, sucrose, sorbitol, glycine, proline, arginine hydrochloride, etc. are selected as stabilizers for a study on stabilizer.

As used herein, "surfactant" is an organic substance with an amphiphilic structure (possessing both hydrophilicity and hydrophobicity), and can be classified into anionic, cationic, and nonionic surfactants. The surfactant includes polysorbate, polyethylene glycol, polypropylene glycol, and the like. In an exemplary embodiment of the present disclosure, polysorbate 20 or polysorbate 80 is selected as the surfactant.

As used herein, "buffer system" is a system comprising one or more buffering agents or buffers and/or acid/base conjugates thereof, particularly comprising one buffering agent or buffer and/or acid/base conjugate thereof. In addition, herein, "buffer" is a compound solution known to be safely applicable in a pharmaceutical formulation and capable of maintaining or controlling the pH value of the pharmaceutical formulation within a desired range. For example, the buffer includes a phosphate buffer, an acetate buffer, a citrate buffer, and certain amino acid buffers (e.g., arginine or histidine). In an exemplary example of the present disclosure, a citric acid-disodium hydrogen phosphate buffer, a citrate buffer, an acetate buffer, and a histidine buffer are selected for a buffer study. The histidine buffer is a buffer containing the amino acid histidine. Examples of the histidine buffer include histidine hydrochloride, histidine acetate, histidine phosphate, and histidine sulfate. However, in some specific embodiments of the present disclosure, the histidine buffer used is a histidine-histidine hydrochloride buffer.

As used herein, "stability" refers to the resistance of antibodies and other active ingredients to chemical degradation and physical changes under given manufacturing, preparation, transportation, and storage conditions. Specifically, the stability of a pharmaceutical formulation refers to the retention of at least 90%, 95%, 98%, 99%, or 99.5% of the initial or reference amount of the antibody or other active ingredient under given manufacturing, preparation, transportation, and/or storage conditions. The amount of antibodies or other active ingredients may be detected or determined using methods recognized in the art, such as ultraviolet spectrophotometry, chromatography, or SDS-PAGE.

As used herein, "clarity" refers to the degree of turbidity of an aqueous solution. If particles are present in the aqueous solution, when light passes through the solution, the solution will cause light scattering and light absorption, and thus the degree of turbidity of the solution is judged based on this phenomenon. For example, in the art, the clarity and visible foreign matter of an aqueous solution sample are detected by adopting visual inspection via a clarity detector, and the degree of opalescence is categorized into clarity, slight opalescence, heavy opalescence, and opacification. Additionally, the turbidity of the aqueous solution is measured by detecting the absorbance at 350 nm, and higher absorbance indicates greater turbidity of the sample.

As used herein, "average protein particle size" or "average hydrated protein particle size" is the diameter of protein (hydrated) particles in an aqueous solution. In the art, dynamic light scattering (DLS) is commonly used to measure the protein particle diameter. Herein, "polydispersity index" represents the distribution of peaks for all particle sizes, and "Pk1%" represents the percentage of a single particle size range within the entire particle size range, indicating the degree of dispersion in the particle size distribution.

As used herein, "pH drift" refers to a change in the pH value of a pharmaceutical formulation after ultrafiltration concentration and buffer exchange compared to the pH value of the initial buffer system. For example, the initial buffer system of the pharmaceutical formulation has a pH value of 5.0, and after the pharmaceutical formulation is subjected to ultrafiltration concentration and buffer exchange, the pH value of the buffer system of the pharmaceutical formulation changes from the initial value of 5.0 to 5.2.

As used herein, "high-temperature accelerated testing" refers to a test in which a pharmaceutical formulation sample is placed in an environment with a temperature higher than ambient temperature to detect changes in the quality of the formulation over a period of time, where the temperature typically includes 30 °C, 40 °C, 50 °C, and the like.

As used herein, "aggregation temperature T_{agg} (also known as thermal aggregation temperature)" refers to the temperature at which protein molecules begin to aggregate during the process of temperature elevation.

As used herein, "freeze-thaw" is a process in which a pharmaceutical formulation sample is placed in an environment with a temperature below 0 °C, and after being completely frozen, the pharmaceutical formulation sample is taken out and then placed at room temperature to undergo thawing. "Freeze-thaw cycle" refers to the number of repetitions of freeze-thaw operation, where the temperature includes -20 °C, -40 °C, -60 °C, -80 °C, and the like.

As used herein, "long-term frozen storage testing" is a test in which a pharmaceutical formulation sample is placed in an environment with a temperature below 0 °C to detect changes in the quality of the formulation over a period of time, where the temperature includes -20 °C, -40 °C, -60 °C, -80 °C, and the like.

As used herein, "charge variants" refer to heterogeneous proteins resulting from differences in net charge and charge distribution on the surface of the protein and are generally classified into acidic variants and basic variants. These variants exhibit different chromatographic and electrophoretic behaviors from those of the main component. When a pharmaceutical formulation is examined by cation exchange chromatography (CEX), the main component usually appears as the main peak with a relatively high content, acidic variants appear as acidic peaks eluting earlier than the main peak, and basic variants appear as basic peaks eluting later than the main peak. Additionally, in the art, charge variants are also detected by imaged capillary isoelectric focusing electrophoresis (icIEF).

As used herein, "treatment" is a method for obtaining beneficial or desired outcomes (including clinical outcomes). Obtaining beneficial or desired outcomes includes, but is not limited to, one or more of the following: alleviating one or more symptoms caused by a disease, reducing the severity of the disease, stabilizing the disease (e.g., preventing or delaying disease worsening), delaying or slowing the progression of the disease, improving the state of the disease, increasing or improving quality of life, promoting body weight gain, and/or extending survival. "Treatment" also encompasses a reduction in pathological consequences of cancer.

As used herein, "effective dose" or "effective amount" refers to an amount that is capable of producing a therapeutic effect in an individual upon each administration. This dose may vary depending on a variety of factors, such as the treatment objective, the treatment frequency, the body weight and tolerance of the individual, the severity of the symptoms, the risk of side effects, and the administration route.

As used herein, "about" or "approximately" refers to a variation of a particular value within an acceptable error range that can be determined by those of ordinary skill in the art, and the range will depend in part on how the particular value is measured and determined, i.e., it is limited to the measurement system or measurement tool. For example, in the examples herein, "about" may indicate a range of within 10%, 5%, or 1% of the given value.

### Pharmaceutical Formulation

The present disclosure relates to a pharmaceutical formulation, in particular to a pharmaceutical formulation of an anti-TIGIT antibody, which comprises an anti-TIGIT antibody, a buffer, a stabilizer, a surfactant, and the like.

The pharmaceutical formulation involved in some specific embodiments of the present disclosure comprises an anti-TIGIT antibody having a concentration of 15-25 mg/mL. In some specific embodiments of the present disclosure, the anti-TIGIT antibody has a concentration of 16-24 mg/mL, 17-23 mg/mL, 18-22 mg/mL, or 19-21 mg/mL. In an exemplary embodiment of the present disclosure, the anti-TIGIT antibody has a concentration of 20 mg/mL.

In some specific embodiments of the present disclosure, the buffer is an acetate buffer or a histidine buffer; in certain embodiments of the present disclosure, the buffer is an acetate buffer, while in certain other embodiments of the present disclosure, the buffer is a histidine buffer. In some specific embodiments of the present disclosure, the buffer has a concentration in the range of 5-30 mM. In certain specific embodiments of the present disclosure, the buffer has a concentration of 10-25 mM; in a preferred embodiment of the present disclosure, the buffer has a concentration of 5-15 mM. In certain specific embodiments of the present disclosure, the buffer has a concentration of 5 mM, 10 mM, or 15 mM. In an exemplary embodiment of the present disclosure, the buffer is an acetate buffer having a concentration of 5-15 mM, and in certain embodiments, the buffer is an acetate buffer having a concentration of 5 mM, 10 mM, or 15 mM. In other exemplary embodiments of the present disclosure, the buffer is a histidine buffer having a concentration of 5-15 mM, and in certain embodiments, the buffer is a histidine buffer having a concentration of 5 mM, 10 mM, or 15 mM.

In some specific embodiments of the present disclosure, the stabilizer is sucrose, sorbitol, or proline; in one exemplary embodiment of the present disclosure, the stabilizer is sorbitol; in another exemplary embodiment of the present disclosure, the stabilizer is proline; in one exemplary embodiment of the present disclosure, the stabilizer is sucrose. Regarding the stabilizers, the stabilizer of the pharmaceutical formulation involved in some specific embodiments of the present disclosure has a concentration of 2%-6% (mass-to-volume ratio), and in certain specific embodiments of the present disclosure, the stabilizer has a concentration of 2%, 3%, 4%, 5%, or 6%. In an exemplary embodiment of the present disclosure, the stabilizer is sucrose having a concentration of 2%-6%, and in certain embodiments, the sucrose has a concentration of 2%, 4%, or 6%.

In some specific embodiments of the present disclosure, the surfactant is polysorbate 20 or polysorbate 80. In an exemplary embodiment of the present disclosure, the surfactant is polysorbate 20; in other exemplary embodiments of the present disclosure, the surfactant is polysorbate 80. In certain embodiments of the present disclosure, the surfactant has a concentration of 0.01%-0.05% (mass-to-volume ratio), and in some exemplary embodiments of the present disclosure, the surfactant has a concentration of 0.02%, 0.03%, or 0.05%. In some exemplary embodiments of the present disclosure, the surfactant is polysorbate 20 having a concentration of 0.02%, 0.03%, or 0.05%. In some preferred exemplary embodiments of the present disclosure, the surfactant is polysorbate 80 having a concentration of 0.02%, 0.03%, or 0.05%, and more specifically, in a preferred exemplary embodiment of the present disclosure, the concentration is 0.03% or 0.05%.

The pharmaceutical formulation involved in some specific embodiments of the present disclosure comprises an anti-TIGIT antibody, a histidine or acetate buffer, a stabilizer, and a surfactant, and has a pH value in the range of 5.1-5.7, such as a pH value of 5.1, 5.2, 5.3, 5.4, 5.5, 5.6, or 5.7, while in some exemplary embodiments of the present disclosure, the pH is 5.1, 5.3, 5.4, or 5.7.

Certain embodiments of the pharmaceutical formulation involved in the present disclosure are preferred, and the preferred embodiments are listed below:
1) A pharmaceutical formulation, comprising the following components:
   (i) an anti-TIGIT antibody, wherein the anti-TIGIT antibody is a single-domain antibody, and a heavy chain variable region of the anti-TIGIT antibody comprises or consists of an amino acid sequence set forth in SEQ ID NO: 1;
   (ii) an acetate buffer or a histidine buffer;
   (iii) sucrose, sorbitol, or proline; and
   (iv) polysorbate 20 or polysorbate 80;
   wherein the pharmaceutical formulation has a pH value of 5.1 to 5.7.
2) The pharmaceutical formulation has a pH value of 5.1, 5.3, 5.4, or 5.7, preferably 5.3 or 5.4, and most preferably 5.4.
3) The buffer has a concentration of 5-15 mM, preferably 10 mM or 15 mM, and most preferably 10 mM.
4) The component (iii) is sucrose.
5) The component (iii) has a concentration of 2%-6%, preferably 2%, 4%, or 6%, and more preferably 2%.
6) The component (iv) is polysorbate 80.
7) The component (iv) has a concentration of 0.01%-0.05%, preferably 0.03% or 0.05%, and more preferably 0.03%.
8) The anti-TIGIT antibody has a concentration of 15-25 mg/mL, preferably 20 mg/mL.
9) A full-length heavy chain of the anti-TIGIT antibody comprises or consists of an amino acid sequence set forth in SEQ ID NO: 2.
10) A pharmaceutical formulation, comprising the following components:
   an anti-TIGIT antibody having a concentration of 15-25 mg/mL;
   a histidine buffer having a concentration of 5-15 mM;
   sucrose having a concentration of 2%-6%; and
   polysorbate 80 having a concentration of 0.01%-0.05%;
   wherein the pharmaceutical formulation has a pH value of 5.1 to 5.7.
11) A pharmaceutical formulation, comprising the following components:
   an anti-TIGIT antibody having a concentration of 20 mg/mL;
   a histidine buffer having a concentration of 10 mM;
   sucrose having a concentration of 2%; and
   polysorbate 80 having a concentration of 0.03%;
   wherein the pharmaceutical formulation has a pH value of 5.4.

### Use and Method

The present disclosure relates to a method for reducing tumor burden in a patient or treating a tumor in a patient, which comprises administering to the patient an effective dose of the pharmaceutical formulation described above. Additionally, the present disclosure relates to use of the pharmaceutical formulation described above in manufacture of a medicament for treating a cancer. More specifically, the method for reducing tumor burden in a patient or the method and use for treating a tumor in a patient involved in the present disclosure comprises administering to the patient an effective dose of the pharmaceutical formulation described above alone or in combination with an additional agent.

In certain embodiments of the present disclosure, the pharmaceutical formulation described above is administered in combination with an additional agent for reducing tumor burden in a patient or treating a tumor in a patient, wherein the additional agent may be a growth inhibitor, a cytotoxic agent, a radiotherapy agent, a protein drug, an immunomodulator, a medicinal nutritional supplement, etc., including, for example, cisplatin, carboplatin, paclitaxel, docetaxel, gefitinib, irinotecan, afatinib, gemcitabine, an anti-VEGF antibody (e.g., bevacizumab), an anti-PD-L1 antibody, an anti-HER2 antibody, an anti-c-Met antibody, an anti-BCMA antibody, an anti-PD1 antibody, an anti-CTLA4 antibody, an anti-LAG-3 antibody, an anti-2B4 antibody, etc.; in a specific embodiment of the present disclosure, the pharmaceutical formulation of the anti-TIGIT antibody described above is administered in combination with an anti-PD-L1 antibody agent; in a specific embodiment of the present disclosure, the pharmaceutical formulation of the anti-TIGIT antibody described above is administered in combination with an anti-PD-1 antibody agent.

In certain embodiments of the present disclosure, the tumor or the cancer is a solid tumor; in certain other embodiments of the present disclosure, the tumor or the cancer is a hematological tumor (e.g., leukemia). In certain embodiments, the tumor or the cancer is selected from the group consisting of mesothelioma, lung cancer, pancreatic cancer, ovarian cancer, breast cancer, colon cancer, pleural tumor, glioblastoma, esophageal cancer, synovial sarcoma, thymic carcinoma, cervical cancer, endometrial cancer, melanoma, gastric tumor, cholangiocarcinoma, head and neck cancer, lymphoma, hematological cancer, and a combination thereof. More specifically, in certain embodiments of the present disclosure, the tumor or the cancer is lung cancer; in certain embodiments of the present disclosure, the tumor or the cancer is gastric cancer; in certain embodiments of the present disclosure, the tumor or the cancer is melanoma; in certain embodiments of the present disclosure, the tumor or the cancer is lymphoma; in certain embodiments of the present disclosure, the tumor or the cancer is cervical cancer.

The present disclosure provides a drug delivery device (such as a syringe or a pre-filled syringe) comprising the pharmaceutical formulation described above. Through the drug delivery device, the pharmaceutical formulation comprising the anti-TIGIT antibody described above is administered to a patient, so as to treat or ameliorate the symptoms of a cancer or tumor in the patient.

The stability of pharmaceutical formulations can be assessed by methods conventional in the art, including light scattering detection, size exclusion chromatography detection, and the like. Other methods for testing the stability of pharmaceutical formulations used in the present disclosure are also well-known in the art. The sample detection methods used in the specific embodiments of the present disclosure are described below.

Additionally, the present application also relates to the following embodiments:
1. A pharmaceutical formulation, comprising:
   (i) an anti-TIGIT antibody, wherein the anti-TIGIT antibody is a single-domain antibody, and a heavy chain variable region of the anti-TIGIT antibody comprises or consists of an amino acid sequence of SEQ ID NO: 1;
   (ii) 5-15 mM histidine buffer;
   (iii) 2% (w/v)-6% (w/v) sucrose; and
   (iv) 0.01% (w/v)-0.05% (w/v) polysorbate 80;
   wherein the pharmaceutical formulation has a pH value of 5.1 to 5.7.
2. The pharmaceutical formulation according to embodiment 1, wherein the anti-TIGIT antibody has a concentration of 15-25 mg/mL.
3. The pharmaceutical formulation according to embodiment 1 or 2, wherein the anti-TIGIT antibody has a concentration of 20 mg/mL.
4. The pharmaceutical formulation according to any one of embodiments 1-3, wherein a full-length heavy chain of the anti-TIGIT antibody comprises or consists of an amino acid sequence of SEQ ID NO: 2.
5. The pharmaceutical formulation according to any one of embodiments 1-4, comprising:
   the anti-TIGIT antibody at 20 mg/mL;
   10 mM histidine buffer;
   2% (w/v) sucrose; and
   0.03% (w/v) polysorbate 80;
   wherein the pharmaceutical formulation has a pH value of 5.1 to 5.7.
6. The pharmaceutical formulation according to any one of embodiments 1-5, wherein the pharmaceutical formulation preferably has a pH value of 5.4.
7. The pharmaceutical formulation according to any one of embodiments 1-6 for use in treating a cancer in a subject.
8. The pharmaceutical formulation for use according to embodiment 7, wherein the cancer is a solid tumor or a hematological tumor.
9. The pharmaceutical formulation for use according to embodiment 7 or 8, wherein the cancer is selected from the group consisting of mesothelioma, lung cancer, pancreatic cancer, ovarian cancer, breast cancer, colon cancer, pleural tumor, glioblastoma, esophageal cancer, synovial sarcoma, thymic carcinoma, cervical cancer, endometrial cancer, gastric tumor, melanoma, cholangiocarcinoma, head and neck cancer, lymphoma, hematological cancer, and a combination thereof.
10. The pharmaceutical formulation for use according to any one of embodiments 7-9, wherein the pharmaceutical formulation is administered in combination with an additional agent.
11. The pharmaceutical formulation for use according to embodiment 10, wherein the additional agent is an anti-PD-1 antibody and/or an anti-PD-L1 antibody.
12. The pharmaceutical formulation for use according to embodiment 10 or 11, wherein the additional agent is an anti-PD-1 antibody.
13. Use of the pharmaceutical formulation according to any one of embodiments 1-6 in manufacture of a medicament for treating a cancer in a subject.
14. The use according to embodiment 13, wherein the cancer is a solid tumor or a hematological tumor.
15. The use according to embodiment 13 or 14, wherein the cancer is selected from the group consisting of mesothelioma, lung cancer, pancreatic cancer, ovarian cancer, breast cancer, colon cancer, pleural tumor, glioblastoma, esophageal cancer, synovial sarcoma, thymic carcinoma, cervical cancer, endometrial cancer, gastric tumor, melanoma, cholangiocarcinoma, head and neck cancer, lymphoma, hematological cancer, and a combination thereof.
16. The use according to any one of embodiments 13-15, wherein the pharmaceutical formulation is administered in combination with an additional agent.
17. The use according to embodiment 16, wherein the additional agent is an anti-PD-1 antibody and/or an anti-PD-L1 antibody.
18. The use according to embodiment 16 or 17, wherein the additional agent is an anti-PD-1 antibody.
19. A method for reducing tumor burden in a patient and/or treating a tumor in a patient, comprising administering to the patient an effective dose of the pharmaceutical formulation according to any one of embodiments 1-6.
20. The method according to embodiment 19, wherein the tumor is a solid tumor or a hematological tumor.
21. The method according to embodiment 19 or 20, wherein the tumor is selected from the group consisting of mesothelioma, lung cancer, pancreatic cancer, ovarian cancer, breast cancer, colon cancer, pleural tumor, glioblastoma, esophageal cancer, synovial sarcoma, thymic carcinoma, cervical cancer, endometrial cancer, gastric tumor, cholangiocarcinoma, head and neck cancer, lymphoma, hematological cancer, and a combination thereof.
22. The method according to any one of embodiments 19-21, wherein the pharmaceutical formulation is administered in combination with an additional agent.
23. The method according to embodiment 22, wherein the additional agent is an anti-PD-1 antibody and/or an anti-PD-L1 antibody.
24. The method according to embodiment 22 or 23, wherein the additional agent is an anti-PD-1 antibody.
25. A drug delivery device, comprising the pharmaceutical formulation according to any one of embodiments 1-6.

### Examples

### Sample detection methods

### 1. Appearance and visible foreign matter detection

The appearance of a sample includes color, clarity, visible foreign matter, and the like. The specific procedures were as follows: After the sample bottle was cleaned, the appearance of the liquid in the bottle was observed using a water-filled sample bottle as a negative control. First, in a darkroom, the illuminance of a clarity detector was adjusted to 1000-3750 Lux, and then the sample bottle was placed at the edge of the clarity detector's light-shielding plate (25 cm). The clarity and visible foreign matter of the sample were examined by visual inspection against a black background. Then, under daylight lamp illumination, the color of the sample was observed by visual inspection against a white background.

### 2. Dynamic light scattering (DLS) detection

The average protein particle size and polydispersity index of the samples were measured using a Wyatt DynaPro Plate Reader-III high-throughput dynamic/static light scattering instrument, and PK1% was calculated. The specific procedures were as follows: 25 µL of the sample was taken and added to the microwells of a 384-well plate in a superclean bench. After being coated with a film, the 384-well microplate was centrifuged at 2000 rpm for 2 min, and then the average protein particle size of the sample in the 384-well plate was measured using the Wyatt DynaPro Plate Reader-III high-throughput dynamic/static light scattering instrument. The detailed detection parameter settings are shown in Table 1.

**Table 1. DLS detection parameter settings**

| **Module** | **Parameter** | **Set value** |
|---|---|---|
| Experiment | Experiment Type | Isothermal |
| | Enable auto-attenuation | Yes |
| | Image of each well | Yes |
| | DLS acquisition time | 5s |
| | DLS acquisitions per measurement | 10 |
| | Measure SLS | No |
| Temperature | Start temperature | 25 °C |
| | Plate sealant | Sealing tape |
| End of experiment | Set temperature | 25 °C |
| | Laser on | No |

### 3. Size exclusion chromatography (SEC) detection

Size exclusion chromatography (SEC) detection was performed using an Agilent 1260 high-performance liquid chromatograph. The detection parameters are shown in Table 2. Before testing, the formulation samples were diluted to 1.0 mg/mL using the mobile phase.

**Table 2. SEC detection parameter settings**

| **Parameter** | **Set value** |
|---|---|
| Flow rate, mL/min | 0.5 |
| Column temperature | Room temperature |
| Sample injection volume, µg | 50.0 |
| Chromatographic column model | TOSOH TSKgel G3000(7.8 mm×300 mm, 5 µm) |
| Detection wavelength, nm | 280 |
| Mobile phase composition | 100 mmol/L phosphate buffer, 300 mmol/L sodium chloride, pH value of 6.8 |
| Elution method | Isocratic elution |
| Elution time, min | 30 |
| Temperature of sample injection bottle, °C | 6~8 |

### 4. Dynamic particle morphology analysis (FlowCam)

Dynamic particle morphology analysis was performed using the FlowCam 8100 particle analyzer to determine the morphology and quantity of subvisible particles in the samples. The specific detection parameter settings are shown in Table 3. The samples were manually injected at 250 µL, and each sample was tested once.

**Table 3. FlowCam instrument detection parameter settings**

| **Category** | **Parameter** | **Set value** |
|---|---|---|
| Capture | Minimum distance between particles (µm) | 0 |
| | Fill gaps in images | 2 |
| Detection threshold | Dark pixels | 15 |
| | Bright pixels | 15 |
| | Include both bright and dark pixels | Selected |
| Flow cell | Flow cell type | FC80FV |
| | Flow cell depth, µm | 81 |
| | Flow cell width, µm | 700 |
| Fluidics | Sample injection volume, mL | 0.2 |
| | Flow rate, mL/min | 0.1 |
| | Detection efficiency | About 70% |

### 5. Detection for absorbance at 350 nm (A₃₅₀)

The absorbance detection was performed using the SpectraMax^{®} 190 light absorption microplate reader (hereinafter referred to as the "microplate reader") to measure the turbidity of the samples. The specific procedures were as follows: 100 µL of the sample was taken and added to a 96-well plate, and then the absorbance at 350 nm of the sample in the 96-well plate was measured using the microplate reader.

### 6. Preparation of antibody pharmaceutical formulations

The anti-TIGIT antibody involved in the present disclosure is a single-domain antibody. As shown in the table below, the amino acid sequence of the heavy chain variable region (VHH) of the anti-TIGIT antibody is set forth in SEQ ID NO: 1, and the amino acid sequence of the full-length heavy chain of the anti-TIGIT antibody is set forth in SEQ ID NO: 2. The anti-TIGIT antibody can be obtained using techniques well known in the art, for example, genetic engineering for production in mammalian host cells, protein synthesis techniques, or other antibody production techniques familiar in the art.

### Anti-TIGIT antibody sequences

| SEQ ID NO: | Name | Amino acid sequence |
|---|---|---|
| 1 | Heavy chain variable region (VHH) of anti-TIGIT antibody | QVQLVESGGGVVQPGRSLRLSCAASGGSFSSYPMGWFRQAPGKERESVA AISSDLRFTYYADSVKGRFTISRDNSKNTLYLQMNSLRAEDTAVYYCAAR TSGVGYYSRAQWYDYWGQGTLVTVSS |
| 2 | Full-length heavy chain of anti-TIGIT antibody | QVQLVESGGGVVQPGRSLRLSCAASGGSFSSYPMGWFRQAPGKERESVA AISSDLRFTYYADSVKGRFTISRDNSKNTLYLQMNSLRAEDTAVYYCAAR TSGVGYYSRAQWYDYWGQGTLVTVSSEPKSCDKTHTCPPCPAPELLGGP SVFLFPPKPKDTLMISRTPEVTCVVVDVSHEDPEVKFNWYVDGVEVHNA KTKPREEQYNSTYRVVSVLTVLHQDWLNGKEYKCKVSNKALPAPIEKTIS KAKGQPREPQVYTLPPSREEMTKNQVSLTCLVKGFYPSDIAVEWESNGQP ENNYKTTPPVLDSDGSFFLYSKLTVDKSRWQQGNVFSCSVMHEALHNHY TQKSLSLSPGK |

The purified anti-TIGIT antibody protein was subjected to ultrafiltration concentration, desalting and buffer exchange, and protein concentration adjustment. Subsequently, stabilizers, surfactants, and buffers were added according to the experimental protocol to prepare the antibody pharmaceutical formulations used for testing and detection in the following examples.

The following examples were conducted to study the pharmaceutical formulations of the anti-TIGIT antibody, including pH value, buffer, surfactant, stabilizer, etc., so as to identify pharmaceutical formulations capable of maintaining the stability of the anti-TIGIT antibody.

All reagents involved in the examples of the present disclosure are commercially available products.

### Example 1. Study on pH Value

The pH value is a primary factor affecting protein stability, as protein stability performance varies at different pH values. In this example, the effects of different pH values on the stability of the anti-TIGIT antibody protein were investigated through the high-temperature (50 °C) accelerated testing to determine the pH range conducive to the stability of the anti-TIGIT antibody. As shown in Table 4, 5 candidate formulations were designed by adopting the single-factor experimental design, with selected pH values of 4.5, 5.0, 5.5, 6.0, and 6.5, respectively. Each formulation contained a citric acid-disodium hydrogen phosphate buffer and polysorbate 20 (also known as PS20 or Tween 20) having a concentration of 0.02%, with a buffer concentration of 15 mM and a protein concentration of 2.0 mg/mL.

**Table 4. Candidate formulation information for screening study on the pH value**

| Formulation No. | pH value | Buffer system | Buffer concentration mM | Protein concentration mg/mL | Surfactant |
|---|---|---|---|---|---|
| CP45 | 4.5 | Citric acid-disodium hydrogen phosphate buffer | 15 | 2.0 | 0.02% polysorbate 20 |
| CP50 | 5.0 | | | | |
| CP55 | 5.5 | | | | |
| CP60 | 6.0 | | | | |
| CP65 | 6.5 | | | | |

The study results showed that: on day 0, there were no significant differences in appearance, protein concentration, initial average protein particle size, and charge variant (CEX) among the formulations with different pH values. The initial protein aggregation temperatures for the formulations were ranked as: CP55 (58.0 °C) ≈ CP50 (57.8 °C) > CP60 (56.2 °C) > CP45 (54.9 °C) > CP65 (51.5 °C), indicating superior protein thermostability at pH 5.0 and pH 5.5. The SEC aggregate content gradually increased as the pH value increased, indicating a greater propensity for proteins to aggregate at higher pH.

After 6 days of standing at 50 °C, the appearance results showed that: At pH 5.0-5.5, the sample was slightly opalescent and had no particles visible to the naked eye; at pH 4.5, the sample was heavily opalescent; when pH ≥ 6.0, the sample had particles visible to the naked eye. The results for the absorbance values of turbidity (A350) indicated that the formulations at pH 4.5 and pH ≥ 6.0 generated large-sized particles during the 50 °C accelerated testing. Consequently, based on the appearance and turbidity detection results, pH 4.5 and pH ≥ 6.0 can be excluded. The DLS results showed that after 6 days of standing at 50 °C, the average protein particle size of the formulation at pH 5.5 (58.0 nm) was larger than the average protein particle size of the formulation at pH 5.0 (44.1 nm), indicating that within the pH range of 5.0-5.5, the lower the pH, the smaller the average protein particle size, which can better alleviate protein aggregation and enhance protein colloidal stability. As shown in FIG. 1a, the SEC results showed that the aggregate increases for the formulations were ranked as: CP45 (0.0%) < CP50 (0.3%) < CP55 (0.7%) < CP60 (1.2%) < CP65 (1.6%), indicating that the higher the pH, the greater the SEC aggregate increase.

Protein degradation and charge variant changes in the 50 °C accelerated testing were investigated by SDS-PAGE and CEX. The SDS-PAGE results showed that the formulation at pH ≥ 6.0 had more fragments, indicating that the anti-TIGIT antibody was more prone to degradation in the formulation at pH ≥ 6.0. As shown in FIG. 1b, the CEX results showed that decreases in main peak and basic peak contents occurred along with an increase in acidic peak content. The acidic peak increases were ranked as: CP65 (12.0%) > CP50 (11.2%) ≈ CP55 (10.9%) ≈ CP60 (10.3%) > CP45 (8.4%), indicating a greater increase in the acidic peak of the formulation at pH 6.5. Consequently, pH 6.5 was excluded. In conclusion, the pH of the pharmaceutical formulation of the anti-TIGIT antibody should fall in the range of about 5.0 to about 5.5.

### Example 2. Study on Buffer System

Buffer systems are often used to maintain the pH of a solution so as to maintain protein stability. The buffer systems used in this example included an acetate buffer, a histidine buffer (a histidine-histidine hydrochloride buffer), a citrate buffer (a citric acid-sodium citrate buffer), and a citric acid-disodium hydrogen phosphate buffer. The buffer system screening study preliminarily investigated the effects of different buffer systems on the stability of the anti-TIGIT antibody protein through the high-temperature (50 °C) accelerated testing. As shown in Table 5, 4 candidate formulations were designed using the single-factor approach. The buffer concentration was 15 mM, the pH value was 5.5, and the surfactant was polysorbate 20 with a concentration of 0.02%.

**Table 5. Candidate formulation information for screening study on the buffers of anti-TIGIT antibody formulations**

| No. | Buffer system | pH value | Buffer concentration mM | Protein concentration mg/mL | Surfactant |
|---|---|---|---|---|---|
| CP55 | Citric acid-disodium hydrogen phosphate buffer | 5.5 | 15 | 2.0 | 0.02% polysorbate 20 |
| C55 | Citrate buffer | | | | |
| A55 | Acetate buffer | | | | |
| H55 | Histidine buffer | | | | |

The study results showed that: on day 0, there were no significant differences in appearance, protein concentration, pH, and molecular variants (SEC) among the different formulations. The initial protein aggregation temperatures for the formulations were ranked as: A55 (61.5 °C) ≈ H55 (61.2 °C) > CP55 (58.0 °C) > C55 (57.1 °C), indicating that the formulations with the acetate buffer and the histidine buffer exhibited the highest initial protein aggregation temperatures and the best thermostability. The DLS results showed that the initial average protein particle sizes in the acetate buffer and the histidine buffer (both 8.2 nm) were smaller than those in the citric acid-disodium hydrogen phosphate buffer and the citrate buffer (9.1 nm and 9.0 nm, respectively), indicating that the protein colloidal stability was good in the acetate buffer and the histidine buffer.

After 6 days of standing at 50 °C, the appearance detection results showed that the formulation with the citrate buffer generated particles visible to the naked eye, while other formulations showed no particles visible to the naked eye and maintained a good appearance. The results for the absorbance values of turbidity (A350) indicated that the formulation with the citrate buffer had the highest absorbance (0.089), indicating that the anti-TIGIT antibody developed large-sized particles in the formulation with the citrate buffer during the 50 °C accelerated testing. Consequently, the citrate buffer was excluded. The DLS results showed that after 6 days of standing at 50 °C, the average protein particle sizes were ranked as: CP55 (58.0 nm) > C55 (20.2 nm) > A55 (9.1 nm) ≈ H55 (9.5 nm), indicating that the formulations with the citric acid-disodium hydrogen phosphate buffer and the citrate buffer generated more sub-micron particles (<1 µm) during the 50 °C accelerated testing. Consequently, the citric acid-disodium hydrogen phosphate buffer was excluded. As shown in FIG. 2a, the SEC aggregate increases for the formulations were ranked as: C55 (0.9%) > CP55 (0.7%) > A55 (0.2%) > H55 (0.0%), indicating that the formulations with the buffer systems of acetate buffer and histidine buffer had the least SEC aggregate increase. Consequently, the acetate buffer and the histidine buffer are the suitable buffer choices. The SDS-PAGE and CEX results (as shown in FIG. 2b) showed that there were no significant differences in degradation trends or charge variant changes of the anti-TIGIT antibody among the 4 buffer formulations. In conclusion, the acetate buffer or histidine buffer should be selected as the buffer for the pharmaceutical formulations comprising the anti-TIGIT antibody.

### Example 3. Study on Buffer System Concentration

The concentration of a buffer system affects both the buffering capacity of the buffer system and the protein stability. Generally, an increase in the concentration of the buffer system may increase the buffering capacity of the buffer system, but may decrease the protein stability, so it is necessary to seek a balance between the two. This example preliminarily investigated the effects of the buffer concentration on the stability of the anti-TIGIT antibody through the high-temperature (50 °C) accelerated testing. In this example, a histidine buffer, polysorbate 20 (PS20) with a concentration of 0.02%, and a pH of 5.5 were selected for each formulation. Three candidate formulations were designed using the single-factor approach, with a buffer concentration ranging from 5-30 mM (mmol/L) (as shown in Table 6) and a protein concentration of 2 mg/mL.

**Table 6. Candidate formulation information for screening study on the buffer concentration of anti-TIGIT antibody formulations**

| Formulation No. | Buffer concentration (mM) | Buffer system | Protein concentration (mg/mL) | pH value | Surfactant |
|---|---|---|---|---|---|
| F-5mM | 5 | Histidine buffer | 2.0 | 5.5 | 0.02% polysorbate |
| F-15mM | 15 | | | | |
| F-30mM | 30 | | | | |

The study results indicated that: on day 0, there were no significant differences in appearance, protein concentration, pH, and molecular variants (SEC) among the formulations with different buffer concentrations. The initial protein aggregation temperatures for the formulations were ranked as: F-5mM (64.6 °C) > F-15mM (61.2 °C) > F-30mM (58.8 °C), indicating that the lower the buffer concentration, the higher the initial aggregation temperature and the better the thermostability. The DLS results showed that the initial average protein particle size of the formulation with the buffer concentration of 5 mM (7.2 nm) was smaller than that of the formulation with the buffer concentration of 15 mM and that of the formulation with a buffer concentration of 30 mM (both 8.2 nm), indicating that compared to those with the high buffer concentrations, the formulation with the low buffer concentration had a smaller average protein particle size and higher protein colloidal stability.

After 6 days of standing at 50 °C, the appearance detection showed that the formulations with different buffer concentrations had no particles visible to the naked eye and maintained a good appearance, and the absorbance values of turbidity (A350) were ranked as: F-30mM (0.031) > F-15mM (0.021) > F-5mM (0.013), indicating that the formulation with a higher buffer concentration generated more large-sized particles during the 50 °C accelerated testing. The DLS results showed that after 6 days of standing at 50 °C, the average protein particle sizes were ranked as: F-30mM (24.9 nm) > F-15mM (9.5 nm) > F-5mM (7.6 nm), indicating that the formulation with the buffer concentration of 30 mM generated more sub-micron particles (<1 µm) during the 50 °C accelerated testing. Consequently, the buffer concentration of 30 mM was excluded. As shown in FIG. 3a, the SEC aggregate increases for the formulations were ranked as: F-30mM (0.2%) > F-15mM (0.0%) > F-5mM (-0.3%), indicating that the higher the buffer concentration of the formulation, the greater the aggregate increase. Therefore, the aggregate analysis study showed that the appropriate buffer concentration range for the formulation is 5-15 mM.

After 6 days of standing at 50 °C, the SDS-PAGE results showed that there were no significant differences in protein degradation among the formulations with different buffer concentrations; the SEC results showed that the SEC fragment increases for the formulations were ranked as: F-30mM (0.3%) < F-15mM (0.4%) < F-5mM (0.5%). Although the differences among the formulations were not significant, there was a downward trend in the fragment increase as the buffer concentration increased. Referring to FIG. 3b, the CEX results showed that decreases in main peak and basic peak contents occurred along with an increase in acidic peak content. The acidic peak increases for the formulations were ranked as: F-30mM (12.0%) > F-15mM (11.7%) > F-5mM (10.8%), indicating that there was an upward trend in the acidic peak increase as the buffer concentration in the formulation increased. Therefore, formulations with a buffer concentration of 5-15 mM can exhibit reduced protein degradation and charge heterogeneity. Based on the analysis and retrieval results described above, the lower the buffer concentration, the smaller the initial average protein particle size of the anti-TIGIT antibody in the formulation, the tighter the protein structure, and the higher the thermostability. Therefore, the buffer concentration range of the pharmaceutical formulations comprising the anti-TIGIT antibody is selected as 5-15 mM.

### Example 4. Study on Surfactant

This example preliminarily investigated the effects of surfactants on the stability of the anti-TIGIT antibody through the high-temperature (50 °C) accelerated testing. In this example, a histidine buffer, a pH of 5.5, and a buffer concentration of 15 mM were selected for each formulation to investigate the effects of polysorbate 20 (also known as polysorbate 20, Tween 20, or PS20) and polysorbate 80 (also known as polysorbate 80, Tween 80, or PS80) on the stability of the anti-TIGIT antibody. The information on each formulation is shown in Table 7.

**Table 7. Formulation information for screening study on the surfactants of anti-TIGIT antibody formulations**

| Formulation No. | Surfactant | Buffer system | Protein concentration mg/mL | pH value | Buffer concentration mM |
|---|---|---|---|---|---|
| F-PS20 | 0.02% polysorbate 20 | Histidine buffer | 2.0 | 5.5 | 15 |
| F-PS80 | 0.02% polysorbate 80 | | | | |

The study results showed that: on day 0, there were no significant differences in appearance, protein concentration, pH, initial average protein particle size, molecular variant (SEC), and charge variant (CEX) between the two formulations. The initial protein aggregation temperatures for the two formulations were 61.2 °C and 62.1 °C, respectively, and the F-PS80 formulation had a slightly higher initial protein aggregation temperature and slightly better thermostability.

After 6 days of standing at 50 °C, the appearance detection results showed that the two formulations had no particles visible to the naked eye and maintained a good appearance. The results for the absorbance values of turbidity (A350) showed that the F-PS80 formulation had an absorbance value (0.019) slightly lower than that of the F-PS20 formulation (0.021). As shown in FIGs. 4a and 4b, the DLS results showed that after 6 days of standing at 50 °C, the average protein particle sizes were ranked as: F-PS20 (9.5 nm) > F-PS80 (8.6 nm). Moreover, the F-PS20 formulation protein exhibited multiple peaks (Pk1 89.4%), while the F-PS80 formulation protein maintained a single peak (Pk1 100%), indicating that oligomers were formed in the F-PS20 formulation during the 50 °C accelerated testing, leading to increased average protein particle size and polydisperse particle size distribution.

After 6 days of standing at 50 °C, the SDS-PAGE results showed that there were no significant differences in protein degradation between the two formulations; the SEC results showed identical SEC fragment increases for the two formulations (0.4%). The CEX results showed that decreases in main peak and basic peak contents occurred along with an increase in acidic peak content, with acidic peak increases of 11.7% and 11.4% for F-PS20 and F-PS80 formulations, respectively.

In conclusion, polysorbate 20 and polysorbate 80 are surfactant choices for the pharmaceutical formulation of the anti-TIGIT antibody. However, the polysorbate 80 formulation exhibited slower protein particle size growth and monodisperse particle size distribution, indicating that polysorbate 80 is superior to polysorbate 20 in inhibiting anti-TIGIT antibody protein aggregation.

### Example 5. Study on Stabilizers

This example preliminarily investigated the effects of stabilizers on the stability of the anti-TIGIT antibody through the high-temperature (50 °C) accelerated testing. In this example, a histidine buffer, a pH of 5.5, a buffer concentration of 15 mM, and polysorbate 20 (PS20) with a concentration of 0.02% were selected for each formulation to investigate the effects of sucrose, sorbitol, glycine, proline, arginine hydrochloride, and sodium chloride on the stability of the anti-TIGIT antibody (Table 8).

**Table 8. Formulation information for screening study on the stabilizers of anti-TIGIT antibody formulations**

| Formulation No. | Stabilizer | Buffer system | Protein concentration mg/mL | pH value | Buffer concentration mM | Surfactant |
|---|---|---|---|---|---|---|
| F_{sucrose} | 3.42% sucrose (100 mM) | Histidine buffer | 2.0 | 5.5 | 15 | 0.02% polysorbate 20 |
| F_{sorbitol} | 1.82% sorbitol (100 mM) | | | | | |
| F_{glycine} | 0.75% glycine (100 mM) | | | | | |
| Fₚᵣₒₗᵢₙₑ | 1.15% proline (100 mM) | | | | | |
| F_{arginine} | 1.06% arginine hydrochloride (50 mM) | | | | | |
| F_{NaCl} | 0.30% NaCl (50 mM) | | | | | |

The study results showed that: on day 0, there were no significant differences in appearance, protein concentration, pH, initial average protein particle size, and molecular variant (SEC) among the formulations with different stabilizers. The initial protein aggregation temperatures for the formulations were ranked as: F_{glycine} (62.1 °C) > F_{sucrose} (61.2 °C) ≈ F_{sorbitol} (61.0 °C) ≈ Fₚᵣₒₗᵢₙₑ (60.8 °C) > F_{arginine} (56.7 °C) > F_{NaCl} (54.6 °C). The F_{arginine} and F_{NaCl} formulations had lower initial aggregation temperatures and poorer thermostability. The CEX results showed that the F_{sucrose} formulation exhibited a slightly lower initial acidic peak level (22.9%), while the other formulations exhibited initial acidic peak levels ranging from 24.0%-24.7%.

After 6 days of standing at 50 °C, the appearance results showed that the F_{glycine} formulation generated a small number of particles visible to the naked eye, the F_{arginine} and F_{NaCl} formulations generated a large number of particles visible to the naked eye, and the F_{sucrose}, F_{sorbitol}, and Fₚᵣₒₗᵢₙₑ formulations had no particles visible to the naked eye and maintained a good appearance; the results for the absorbance values of turbidity (A350) indicated that the absorbance values were ranked as: F_{NaCl} (0.197) > F_{arginine} (0.039) > F_{glycine} (0.031) > Fₚᵣₒₗᵢₙₑ (0.022) ≈ F_{sucrose} (0.021) ≈ F_{sorbitol} (0.018), indicating that the F_{NaCl}, F_{arginine}, and F_{glycine} formulations all generated large-sized particles during the 50 °C accelerated testing. Consequently, based on the appearance and turbidity results, NaCl, arginine, and glycine can be excluded. After 6 days of standing at 50 °C, the average protein particle sizes were ranked as: F_{sucrose} (9.5 nm) ≈ F_{sorbitol} (9.2 nm) > Fₚᵣₒₗᵢₙₑ (8.9 nm). Referring to FIG. 5a, the SEC aggregate increases for the formulations were ranked as: F_{NaCl} (0.5%) > F_{arginine} (0.4%) > F_{glycine} (0.1%) ≈ Fₚᵣₒₗᵢₙₑ (-0.1%) ≈ F_{sucrose} (0.0%) ≈ F_{sorbitol} (0.0%), indicating that there was a greater increase in protein aggregates in the F_{NaCl} and F_{arginine} formulations, while there was no significant increase in aggregates in the Fₚᵣₒₗᵢₙₑ, F_{sucrose}, and F_{sorbitol} formulations.

After 6 days of standing at 50 °C, the SDS-PAGE results showed significant protein degradation in the F_{glycine}, F_{arginine}, and F_{NaCl} formulations; the SEC results showed SEC fragment increases of 0.3%-0.5% for all formulations. Referring to FIG. 5b, the CEX results showed that the acidic peak increases for the formulations were ranked as: F_{glycine} (14.1%) > F_{sucrose} (11.7%) ≈ F_{sorbitol} (11.0%) ≈ Fₚᵣₒₗᵢₙₑ (10.5%) > F_{NaCl} (7.9%) ≈ F_{arginine} (7.7%), indicating that the F_{glycine} formulation exhibited the greatest increase in the acid peak component. Consequently, glycine was excluded. Thus, based on the study results for protein degradation and charge heterogeneity, sucrose, sorbitol, and proline are the suitable stabilizer choices.

In conclusion, sucrose, sorbitol, and proline can significantly inhibit the aggregation and degradation of anti-TIGIT antibody proteins and maintain protein stability. Therefore, sucrose, sorbitol, or proline is selected as the stabilizer of the pharmaceutical formulations comprising the anti-TIGIT antibody, with sucrose being the preferred choice.

### Example 6. Screening for pH Value and Stabilizer Concentration

In this example, an acetate buffer was selected, and a 23 full factorial experiment with 1 center point (3 factors at 2 levels) was conducted. As shown in Table 9, the pH, sucrose concentration, and polysorbate 80 concentration determined in the examples described above were chosen as the factors for investigation. Specifically, the pH values were 4.8, 5.3, and 5.8; the sucrose concentrations were 1.70%, 5.85%, and 10.00%; and the polysorbate 80 concentrations were 0.01%, 0.03%, and 0.05%. As shown in Table 10, a total of 9 candidate formulations were set in this example. The basic physicochemical properties and protein stability data for each formulation were investigated through the high-temperature (40 °C) accelerated testing, with the goal of further screening out the preferred pH value, polysorbate 80 concentration, and sucrose concentration for maintaining the stability of the anti-TIGIT antibody in the formulation.

**Table 9. Screening study on the pH, sucrose concentration, and polysorbate concentration of anti-TIGIT antibody formulations-full factorial experiment design factors and levels**

| **Level** | **Factor** | | |
|---|---|---|---|
| | **pH** | **Sucrose content** | **Polysorbate 80 content** |
| -1 | 4.8 | 1.70% | 0.01% |
| 0 | 5.3 | 5.85% | 0.03% |
| +1 | 5.8 | 10.00% | 0.05% |

**Table 10. Formulation information for screening study on the pH, sucrose concentration, and polysorbate concentration of anti-TIGIT antibody formulations**

| **No.** | **pH** | **Sucrose content, %** | **Polysorbate 80 content, %** | **Buffer system** |
|---|---|---|---|---|
| F1 | 4.8 | 10.0 | 0.01 | Acetate buffer |
| F2 | 5.8 | 10.0 | 0.01 | |
| F3 | 5.3 | 5.85 | 0.03 | |
| F4 | 4.8 | 1.7 | 0.01 | |
| F5 | 4.8 | 1.7 | 0.05 | |
| F6 | 5.8 | 10.0 | 0.05 | |
| F7 | 5.8 | 1.7 | 0.01 | |
| F8 | 5.8 | 1.7 | 0.05 | |
| F9 | 4.8 | 10.0 | 0.05 | |
| Note: The buffer concentration was 5 mmol/L; the protein concentration was 20.0 mg/mL. | | | | |

The anti-TIGIT antibody protein was subjected to ultrafiltration concentration, desalting and buffer exchange, and protein concentration adjustment. Subsequently, stabilizers, surfactants, and buffers were added according to the experimental protocol to prepare 9 target formulations. The formulation samples were filtered using 0.22 µm sterile disposable filters in a biosafety cabinet, followed by aseptic dispensing of 0.6 mL aliquots of each sample into 3 mL sterile vials. The vials were sealed with 13 mm rubber stoppers and 13 mm aluminum-plastic combination caps. Each formulation was sampled and tested according to the experimental design shown in Table 11.

**Table 11. Investigation conditions and detection methods for screening study on the pH and auxiliary material concentration of anti-TIGIT antibody formulations**

| **Study content** | **Investigation condition** | **Detection point/detection method** | | |
|---|---|---|---|---|
| | | **0 days** | **12 days** | **28 days** |
| High temperature | 40 °C | X, Y, Z | X | X, Y |
| X: appearance, protein concentration (A₂₈₀), SEC, DLS, CEX, NR CE-SDS | | | | |
| Y: pH (measured value) | | | | |
| Z: T_{agg} | | | | |

The study results indicated that: on day 0, there were no significant differences in appearance, protein concentration, charge variant (CEX), SEC initial fragment content, and intact IgG content among different formulations. There were significant differences in protein aggregation temperature (Tagg), initial average protein particle size, and initial aggregate content among the formulations. As shown in Table 12, after 28 days of standing at 40 °C, significant differences were observed in the SEC aggregate increase, DLS average protein particle size increase, DLS Pk1% decrease (in aggregation), SEC fragment increase, intact IgG decrease (in degradation), and acidic peak increase (in charge heterogeneity) among different formulations.

**Table 12. Summary of key data from screening study on the pH and auxiliary material concentration of anti-TIGIT antibody formulations (40 °C)**

| **Detection item** | | **Investigation time** | **F1** | **F2** | **F3** | **F4** | **F5** | **F6** | **F7** | **F8** | **F9** |
|---|---|---|---|---|---|---|---|---|---|---|---|
| Basic physicochemical detection | T_{agg}°C | | 63.2 | 64.4 | 63.0 | 62.9 | 63.8 | 63.0 | 62.8 | 61.0 | 61.4 |
| | Appearance^{[1]} | 0 days | **/- | **/- | **/- | **/- | **/- | **/- | **/- | **/- | **/- |
| | | 12 days | **/- | **/- | **/- | **/- | **/- | **/- | **/- | **/- | **/- |
| | | 28 days | **/- | **/- | **/- | **/- | **/- | **/- | **/- | **/- | **/- |
| | pH | 0 days | 5.1 | 6.1 | 5.6 | 5.1 | 5.1 | 6.1 | 6.1 | 6.1 | 5.1 |
| | | 28 days | 5.2 | 6.1 | 5.7 | 5.2 | 5.2 | 6.2 | 6.1 | 6.2 | 5.2 |
| pH of buffer | | | 4.8 | 5.8 | 5.3 | 4.8 | 4.8 | 5.8 | 5.8 | 5.8 | 4.8 |
| Change in pH | | | 0.3 | 0.3 | 0.3 | 0.3 | 0.3 | 0.3 | 0.3 | 0.3 | 0.3 |
| DLS | Average particle size nm | 0 days | 3.9 | 6.7 | 4.7 | 3.3 | 3.4 | 6.8 | 5.5 | 5.6 | 4.0 |
| | | 12 days | 4.1 | 7.3 | 4.9 | 3.4 | 3.5 | 7.3 | 5.7 | 5.9 | 4.2 |
| | | 28 days | 5.8 | 8.5 | 5.1 | 3.7 | 3.5 | 7.2 | 6.1 | 6.0 | 4.4 |
| | Pk1% | 0 days | 98.2 | 100.0 | 100.0 | 100.0 | 100.0 | 100.0 | 100.0 | 100.0 | 86.9 |
| | | 12 days | 94.9 | 100.0 | 100.0 | 100.0 | 100.0 | 98.3 | 100.0 | 100.0 | 95.8 |
| | | 28 days | 63.1 | 72.4 | 93.9 | 84.6 | 100.0 | 94.4 | 97.3 | 100.0 | 90.7 |
| | **Change in average particle size** | | **1.9** | **1.8** | **0.4** | **0.4** | **0.1** | **0.4** | **0.6** | **0.4** | **0.4** |
| | **Change in Pk1%** | | **-35.1** | **-27.6** | **-6.1** | **-15.4** | **0.0** | **-5.6** | **-2.7** | **0.0** | **3.8** |
| SEC | Aggregate% | 0 days | 0.4 | 0.7 | 0.5 | 0.4 | 0.5 | 0.7 | 0.8 | 0.8 | 0.5 |
| | | 12 days | 0.5 | 1.7 | 1.0 | 0.5 | 0.6 | 1.9 | 1.9 | 2.0 | 0.6 |
| | | 28 days | 0.8 | 2.5 | 1.4 | 0.8 | 0.8 | 2.7 | 2.8 | 2.8 | 0.9 |
| | Main peak% | 0 days | 99.4 | 99.2 | 99.3 | 99.5 | 99.4 | 99.0 | 99.1 | 99.0 | 99.3 |
| | | 12 days | 99.1 | 97.7 | 98.6 | 99.0 | 98.8 | 97.4 | 97.5 | 97.4 | 98.9 |
| | | 28 days | 98.5 | 96.6 | 97.8 | 98.5 | 98.6 | 96.2 | 96.4 | 96.2 | 98.4 |
| | Fragment% | 0 days | 0.2 | 0.2 | 0.2 | 0.1 | 0.2 | 0.2 | 0.2 | 0.2 | 0.2 |
| | | 12 days | 0.4 | 0.5 | 0.4 | 0.4 | 0.6 | 0.7 | 0.6 | 0.7 | 0.4 |
| | | 28 days | 0.7 | 1.0 | 0.7 | 0.8 | 0.7 | 1.1 | 0.9 | 1.0 | 0.8 |
| | **Change in aggregate (%)** | | **0.4** | **1.8** | **0.9** | **0.4** | **0.3** | **2.0** | **2.0** | **2.0** | **0.4** |
| | **Change in main peak (%)** | | **-0.9** | **-2.6** | **-1.5** | **-1.0** | **-0.8** | **-2.8** | **-2.7** | **-2.8** | **-0.9** |
| | **Change in fragment (%)** | | **0.5** | **0.8** | **0.6** | **0.6** | **0.5** | **0.9** | **0.7** | **0.9** | **0.6** |
| CEX | Acidic peak% | 0 days | 19.9 | 20.2 | 20.7 | 20.2 | 21.0 | 20.4 | 20.7 | 20.7 | 20.7 |
| | | 12 days | 25.0 | 24.2 | 24.5 | 25.2 | 25.3 | 24.7 | 24.6 | 24.9 | 25.7 |
| | | 28 days | 36.1 | 34.8 | 35.0 | 37.2 | 37.6 | 35.2 | 35.6 | 36.8 | 37.4 |
| | Main peak% | 0 days | 50.0 | 49.8 | 49.5 | 50.0 | 49.4 | 50.0 | 49.4 | 50.2 | 50.0 |
| | | 12 days | 46.0 | 46.9 | 46.2 | 45.6 | 45.8 | 47.1 | 47.3 | 47.4 | 46.3 |
| | | 28 days | 44.2 | 46.2 | 45.7 | 43.9 | 43.8 | 46.2 | 46.2 | 45.1 | 43.9 |
| | Basic peak% | 0 days | 30.1 | 30.0 | 29.9 | 29.8 | 29.6 | 29.6 | 29.9 | 29.1 | 29.3 |
| | | 12 days | 29.0 | 28.9 | 29.3 | 29.2 | 28.9 | 28.2 | 28.0 | 27.8 | 28.0 |
| | | 28 days | 19.7 | 19.0 | 19.4 | 18.9 | 18.6 | 18.6 | 18.2 | 18.0 | 18.7 |
| | **Change in acidic peak (%)** | | **16.2** | **14.7** | **14.3** | **17.1** | **16.7** | **14.8** | **15.0** | **16.2** | **16.7** |
| | **Change in main peak (%)** | | **-5.8** | **-3.6** | **-3.8** | **-6.1** | **-5.6** | **-3.8** | **-3.3** | **-5.1** | **-6.1** |
| | **Change in basic peak (%)** | | **-10.4** | **-11.0** | **-10.5** | **-11.0** | **-11.1** | **-11.1** | **-11.7** | **-11.1** | **-10.6** |
| NR CE-SDS | Intact IgG | 0 days | 98.0 | 98.0 | 98.1 | 98.2 | 98.1 | 98.1 | 98.0 | 98.0 | 98.0 |
| | | 12 days | 97.2 | 96.4 | 96.8 | 96.6 | 96.9 | 96.2 | 96.4 | 96.3 | 97.0 |
| | | 28 days | 96.1 | 95.9 | 96.2 | 95.7 | 95.6 | 93.7 | 95.9 | 96.0 | 96.2 |
| | **Change in intact IgG (%)** | | **-1.9** | **-2.1** | **-1.9** | **-2.5** | **-2.5** | **-4.4** | **-2.1** | **-2.0** | **-1.8** |
| Note: [1] Appearance description is divided into two aspects: opalescence and particles. Degree of opalescence: * denotes clarity, ** denotes slight opalescence, *** denotes heavy opalescence, and **** denotes opacification; degree of particles: - denotes no particles visible to the naked eye, and + denotes the presence of particles visible to the naked eye. | | | | | | | | | | | |

The experimental data described above were imported into the JMP^{®} 15.0.0 software, and multiple linear regression and binomial equation fitting were performed on each factor using the standard least squares method. The analysis results are shown in Table 13. The model P values for the initial average protein particle size, measured pH, SEC aggregate increase, DLS average protein particle size increase, and DLS Pk1% decrease were all less than 0.1, indicating that the selected models are reliable and statistically significant. Their adjusted coefficients of determination (R²) were all greater than 0.95, indicating a good fit between the models and the experimental values. Therefore, the regression models can be used to replace the actual test points to analyze and predict the experimental results.

**Table 13. Establishment of models for screening study on the pH and auxiliary material concentration of anti-TIGIT antibody formulations**

| **Response value** | **P value** | **R²** |
|---|---|---|
| Initial average protein particle size | 0.0074 | 1.00 |
| Measured pH | 0.0027 | 1.00 |
| SEC aggregate increase | 0.0366 | 0.99 |
| DLS average protein particle size increase | 0.0912 | 0.97 |
| DLS Pk1% decrease | 0.0575 | 0.98 |
| Initial aggregate content | 0.1223 | 0.96 |
| Aggregation temperature T_{agg} | 0.3571 | 0.86 |
| SEC fragment increase | 0.2666 | 0.90 |
| Intact IgG decrease | 0.4784 | 0.80 |
| Acidic peak increase | 0.6954 | 0.67 |

Referring to Table 14, the model analysis results indicated that the pH value and sucrose concentration had significant positive effects on the initial average protein particle size, that is, the higher the pH value and sucrose concentration, the larger the initial average protein particle size; the pH value had a significant positive effect on the aggregate increase, that is, the higher the pH, the greater the aggregate increase; the polysorbate 80 concentration had a relatively significant negative effect on the DLS average protein particle size increase and Pk1% decrease, that is, the higher the polysorbate 80 concentration, the less the DLS particle size increase and Pk1% decrease, while the sucrose concentration had a relatively significant positive effect on the DLS particle size increase and Pk1% decrease, that is, the higher the sucrose concentration, the greater the DLS particle size increase and Pk1% decrease.

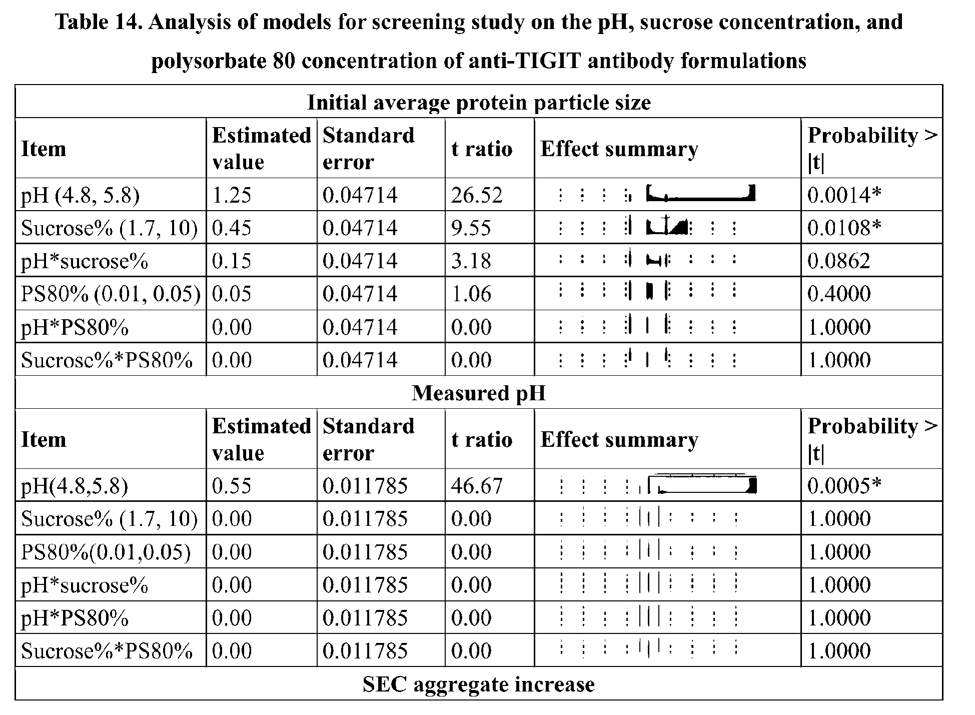

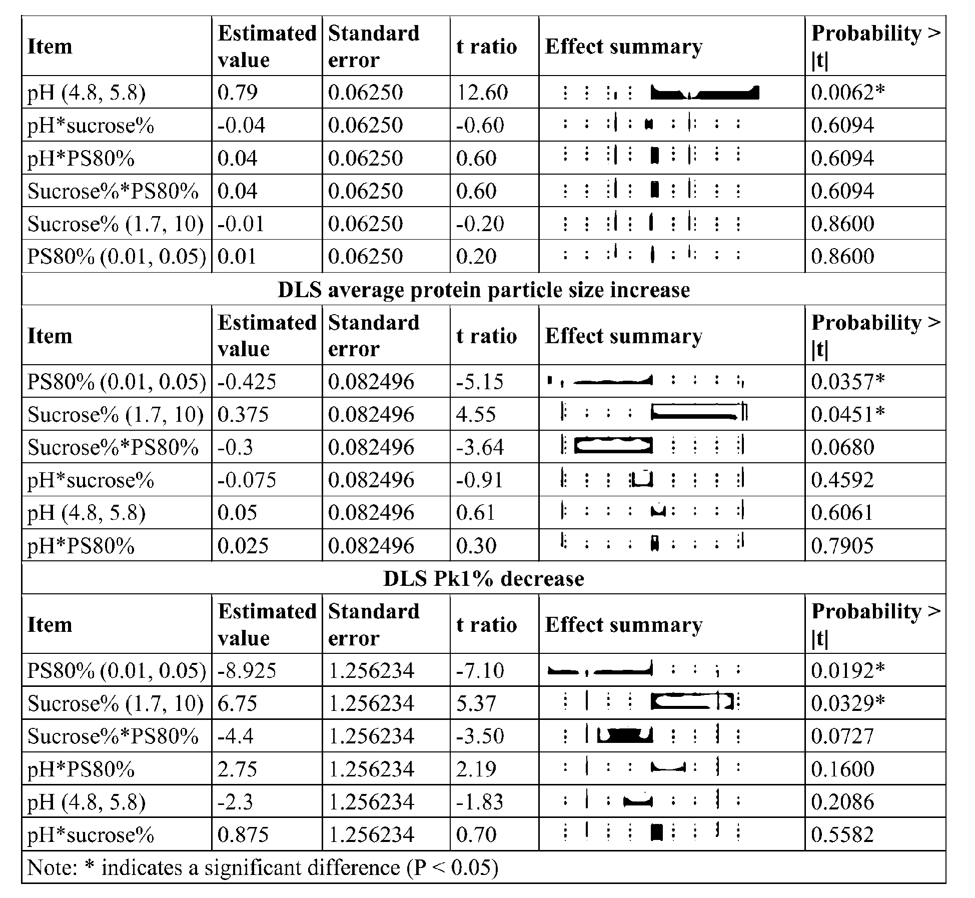

Based on the above experimental results, as shown in Table 15, IMP^{®} 15.0.0 software was used to predict and profile each response factor, and the importance of response factors with model P values less than 0.1 was set to 0.2.

**Table 15. Screening study on the pH, sucrose concentration, and polysorbate 80 concentration of anti-TIGIT antibody formulations-desirability settings**

| **Response** | **Desirability** | **Importance^{[1]}** |
|---|---|---|
| Initial average protein particle size | Minimize | 0.2 |
| Measured pH | Match target value | 0.2 |
| 40 °C for 28 days-SEC aggregate increase | Minimize | 0.2 |
| 40 °C for 28 days-DLS average protein particle size increase | Minimize | 0.2 |
| 40 °C for 28 days-DLS Pk1% decrease | Minimize | 0.2 |
| Note: The sum of importance values for all responses equals 1. | | |

As shown in FIG. 6, the desirability profiler results showed that when the pH was in the range of 4.8-5.1, the formulation desirability gradually increased, reaching its maximum value at pH 5.1 (corresponding to a measured pH of 5.4); when the pH exceeded 5.1 (corresponding to a measured pH of 5.4), the formulation desirability gradually decreased; as the sucrose concentration decreased, the formulation desirability showed an upward trend, approaching its maximum value at a sucrose concentration of 2%; an increase in the polysorbate 80 concentration led to an upward trend in the formulation desirability, however, when the polysorbate 80 concentration was in the range of 0.01%-0.05%, the formulation desirability remained relatively high. In conclusion, for the pharmaceutical formulations comprising the anti-TIGIT antibody, the selected pH range is 4.8-5.4 (corresponding to a measured pH of 5.1-5.7), the selected sucrose concentration range is 2%-6%, and the selected polysorbate 80 concentration range is 0.01%-0.05%. The desirability profiler results showed the preferred pH and component concentrations: a theoretical pH value of 5.1 (i.e., an actual pH of 5.4), a sucrose concentration of 2%, and a polysorbate 80 concentration of 0.03%.

### Example 7. Study on Component Concentration

### 7.1 Study on sucrose concentration

Target formulations were prepared according to the formulation information shown in Table 16, and sampling and testing were carried out according to the experimental design shown in Table 17. The fact that a sucrose concentration range of 2%-6% in the formulation is conducive to the stability of the anti-TIGIT antibody protein was studied and verified through the shaking test and freeze-thaw test.

**Table 16. Formulation information for screening study on the sucrose concentration of anti-TIGIT antibody formulations**

| **No.** | **Sucrose content, %** | **Polysorbate 80 content, %** | **Buffer system** | **Protein concentration, mg/mL** |
|---|---|---|---|---|
| F1 | 2 | 0.03 | 20 mM acetate buffer pH 5.1 | 20.0 |
| F3 | 4 | 0.03 | | |
| F4 | 6 | 0.03 | | |

**Table 17. Investigation conditions and detection methods for screening study on the auxiliary material concentration of anti-TIGIT antibody formulations**

| **Study content** | **Investigation condition** | **Sample of investigation** | **Detection point/detection method** | | |
|---|---|---|---|---|---|
| | | | **0 cycles** | **5 cycles** | **10 cycles** |
| Freeze-thaw | -20 °C-room temperature | F1 | X, Y | X | X, Y |
| | | F3 | | | |
| | | F4 | | | |
| X: appearance, protein concentration (A₂₈₀), SEC, DLS, FlowCam, CEX, NR&R CE-SDS | | | | | |
| Y: pH, osmotic pressure | | | | | |

Before freeze-thaw cycling, there were no significant differences in basic physicochemical properties (appearance, protein concentration, pH), molecular variant (SEC, CE-SDS), charge variant (CEX), and subvisible particle (SVP) level among all formulations. As shown in Table 18, the initial protein particle sizes were ranked as: formulation F1 (6.7 nm) < formulation F3 (6.9 nm) < formulation F4 (7.3 nm). Referring to FIGs. 7a, 7b, 7c, 7d, and 7e, after 10 repeated freeze-thaw cycles under a condition of -20 °C to room temperature, there were no significant changes in the protein appearance, subvisible particle level, and purity (SEC, CEX, and CE-SDS) for each formulation.

**Table 18. Key data table-1 for screening study on the sucrose concentration and polysorbate 80 concentration of anti-TIGIT antibody formulations (freeze-thaw)**

| **Detection item** | | **Number of cycles (times)** | **F1** | **F2** | **F3** | **F4** |
|---|---|---|---|---|---|---|
| | | | **2% sucrose + 0.03% PS80** | **2% sucrose + 0.05% PS80** | **4% sucrose + 0.03% PS80** | **6% sucrose + 0.03% PS80** |
| Basic physicochemical properties | Appearance^{[1]} | 0 | **/- | **/- | **/- | **/- |
| | | 5 | **/- | **/- | **/- | **/- |
| | | 10 | **/- | **/- | **/- | **/- |
| | Protein concentration | 0 | 19.9 | 19.7 | 19.5 | 19.3 |
| | | 5 | 19.3 | 19.6 | 19.8 | 19.5 |
| | | 10 | 19.3 | 20.0 | 19.3 | 19.5 |
| | pH | 0 | 5.3 | 5.3 | 5.3 | 5.3 |
| | | 5 | 5.3 | 5.3 | 5.3 | 5.3 |
| | | 10 | 5.3 | 5.3 | 5.3 | 5.3 |
| | Osmotic pressure | 0 | 97.0 | 99.0 | 161.0 | 232.0 |
| | | 5 | 101.0 | 99.0 | 164.0 | 236.0 |
| | | 10 | 101.0 | 100.0 | 165.0 | 241.0 |
| DLS | Average particle size nm | 0 | 6.7 | 6.7 | 6.9 | 7.3 |
| | | 5 | 6.7 | 7.2 | 6.7 | 6.9 |
| | | 10 | 6.6 | 7.3 | 6.7 | 7.0 |
| | Pk1% | 0 | 100.0 | 100.0 | 100.0 | 100.0 |
| | | 5 | 100.0 | 100.0 | 100.0 | 100.0 |
| | | 10 | 100.0 | 100.0 | 100.0 | 100.0 |
| SEC | Aggregate% | 0 | 0.4 | 0.4 | 0.4 | 0.4 |
| | | 5 | 0.4 | 0.4 | 0.4 | 0.4 |
| | | 10 | 0.4 | 0.4 | 0.4 | 0.4 |
| | Main peak% | 0 | 99.3 | 99.3 | 99.3 | 99.3 |
| | | 5 | 99.4 | 99.3 | 99.4 | 99.3 |
| | | 10 | 99.3 | 99.3 | 99.4 | 99.3 |
| | Fragment% | 0 | 0.3 | 0.3 | 0.3 | 0.3 |
| | | 5 | 0.2 | 0.3 | 0.2 | 0.2 |
| | | 10 | 0.3 | 0.3 | 0.2 | 0.3 |
| | **Change in aggregate%** | | **0.0** | **0.0** | **0.0** | **0.0** |
| | **Change in main peak%** | | **0.0** | **0.0** | **0.1** | **0.0** |
| | **Change in fragment%** | | **0.0** | **0.0** | **-0.1** | **0.0** |
| CEX | Acidic peak% | 0 | 17.8 | 17.8 | 17.7 | 17.8 |
| | | 5 | 17.8 | 17.7 | 17.7 | 17.7 |
| | | 10 | 17.8 | 17.9 | 17.8 | 17.8 |
| | Main peak% | 0 | 51.3 | 51.2 | 51.4 | 51.2 |
| | | 5 | 51.5 | 51.2 | 51.4 | 51.3 |
| | | 10 | 51.3 | 51.0 | 51.3 | 51.2 |
| | Basic peak% | 0 | 31.0 | 31.0 | 30.9 | 31.0 |
| | | 5 | 30.8 | 31.1 | 31.0 | 31.0 |
| | | 10 | 31.0 | 31.1 | 30.9 | 31.0 |
| | **Change in acidic peak%** | | **0.0** | **0.1** | **0.1** | **0.0** |
| | **Change in main peak%** | | **0.0** | **-0.2** | **-0.1** | **0.0** |
| | **Change in basic peak%** | | **0.0** | **0.1** | **0.0** | **0.0** |
| CE-SDS | Intact antibody% | 0 | 99.2 | 99.3 | 99.0 | 99.0 |
| | | 5 | 99.3 | 99.3 | 99.4 | 99.3 |
| | | 10 | 99.2 | 99.2 | 99.2 | 99.2 |
| | Intact heavy chain% | 0 | 97.5 | 97.5 | 97.6 | 97.5 |
| | | 5 | 98.2 | 98.3 | 98.2 | 98.3 |
| | | 10 | 98.4 | 98.3 | 98.3 | 98.3 |
| | **Change in intact antibody%** | | **0.0** | **-0.1** | **0.2** | **0.2** |
| | **Change in intact heavy chain%** | | **0.9** | **0.8** | **0.7** | **0.8** |
| FlowCam particles/mL | 1~2 µm | 0 | 26 | 26 | 26 | 34 |
| | | 3 | 35 | 336 | 226 | 528 |
| | | 5 | 303 | 83 | 69 | 117 |
| | | 10 | 213 | 620 | 155 | 231 |
| | 2~5 µm | 0 | 43 | 60 | 18 | 52 |
| | | 3 | 95 | 471 | 364 | 1021 |
| | | 5 | 572 | 198 | 163 | 309 |
| | | 10 | 587 | 1432 | 422 | 529 |
| | 5~10 µm | 0 | 17 | 26 | 18 | 9 |
| | | 3 | 17 | 92 | 87 | 147 |
| | | 5 | 139 | 99 | 51 | 83 |
| | | 10 | 34 | 114 | 26 | 51 |
| | 10~25 µm | 0 | 17 | 43 | 0 | 43 |
| | | 3 | 35 | 50 | 95 | 78 |
| | | 5 | 35 | 25 | 26 | 42 |
| | | 10 | 43 | 35 | 43 | 43 |
| | 25 µm+ | 0 | 0 | 0 | 0 | 0 |
| | | 3 | 0 | 25 | 9 | 0 |
| | | 5 | 9 | 0 | 0 | 0 |
| | | 10 | 0 | 0 | 0 | 9 |
| Note: [1] Appearance description is divided into two aspects: opalescence and particles. Degree of opalescence: * denotes clarity, ** denotes slight opalescence, *** denotes heavy opalescence, and **** denotes opacification; degree of particles: - denotes no particles visible to the naked eye, and + denotes the presence of particles visible to the naked eye. | | | | | | |

In conclusion, after 10 freeze-thaw cycles under a condition of -20 °C to room temperature, there were no significant changes in the purity of the anti-TIGIT antibody protein and the number of sub-micron and larger particles for each formulation. Therefore, the anti-TIGIT antibody protein exhibited good freeze-thaw stability in formulations with a sucrose concentration range of 2%-6%. Moreover, in view of the initial average protein particle size results and the stabilizer concentration screening study results in Examples 5 and 6, the preferred sucrose concentration is 2%.

### 7.2 Study on polysorbate 80 concentration

Target formulations were prepared according to the formulation information shown in Table 19, and sampling and testing were carried out according to the experimental design shown in Table 20. The fact that a polysorbate 80 concentration range of 0.03%-0.05% in the formulation is conducive to the stability of the anti-TIGIT antibody protein was studied and verified through the shaking test and repeated freeze-thaw test.

**Table 19. Formulation information for screening study on the polysorbate 80 concentration in anti-TIGIT antibody formulations**

| **No.** | **Sample** | **Sucrose content, %** | **Polysorbate 80 content, %** | **Buffer system** | **Protein concentration, mg/mL** |
|---|---|---|---|---|---|
| F1 | PS80-0.03% | 2 | 0.03 | 20 mM acetate buffer pH 5.1 | 20.0 |
| F2 | PS80-0.05% | 2 | 0.05 | | |

**Table 20. Investigation conditions and detection methods for screening study on the polysorbate 80 concentration in anti-TIGIT antibody formulations**

| **Study content** | **Investigation condition** | **Sample of investigation** | **Detection point/detection method** | | |
|---|---|---|---|---|---|
| | | | **0 days** | **14 days** | **28 days** |
| Shaking | 25 °C, 200 rpm | F1 | X, Y | X | X, Y |
| | | F2 | | | |

| **Study content** | **Investigation condition** | **Sample of investigation** | **0 cycles** | **5 cycles** | **10 cycles** |
|---|---|---|---|---|---|
| Freeze-thaw | -20 °C-room temperature | F1 | X, Y | X | X, Y |
| | | F2 | | | |
| X: appearance, protein concentration (A₂₈₀), SEC, DLS, FlowCam, CEX, NR&R CE-SDS | | | | | |
| Y: pH, osmotic pressure | | | | | |

As shown in Table 21, on day 0, there were no significant differences in the basic physicochemical properties (appearance, protein concentration, pH, osmotic pressure), initial protein particle size, molecular variant (SEC, CE-SDS), charge variant (CEX), and subvisible particle (SVP) level between the two formulations; after shaking at 25 °C and 200 rpm for 28 days, the two formulations both had no particles visible to the naked eye and maintained a good appearance; the number of subvisible particles (SVPs) in the formulation with 0.05% polysorbate 80 was slightly less than that in the formulation with 0.03% polysorbate 80, but the difference was not significant. Additionally, there were no significant changes in the average protein particle size and purity (SEC, CEX, and CE-SDS) of the two formulations. Therefore, the analysis results of aggregation, degradation, and charge heterogeneity indicated that the polysorbate 80 concentrations of 0.03% and 0.05% can both maintain the stability of the antibody TIGIT antibody protein.

**Table 21. Key data table-2 for screening study on the polysorbate 80 concentration in anti-TIGIT antibody formulations (freeze-thaw)**

| **Detection item** | | **Investigation time (days)** | **F1** | **F2** |
|---|---|---|---|---|
| | | | **0.03% PS80** | **0.05% PS80** |
| Basic physicochemical properties | Appearance^{[1]} | 0 | **/- | **/- |
| | | 14 | **/- | **/- |
| | | 28 | **/- | **/- |
| | Protein concentration | 0 | 19.9 | 19.7 |
| | | 14 | 19.7 | 19.9 |
| | pH | 28 | 5.3 | 5.3 |
| | | 0 | 5.3 | 5.3 |
| | Osmotic pressure | 14 | 97.0 | 99.0 |
| | | 28 | 99.0 | 100.0 |
| DLS | Average particle size nm | 0 | 6.7 | 6.7 |
| | | 14 | 6.7 | 6.8 |
| | | 28 | 7.0 | 6.9 |
| | Pk1 | 0 | 100.0 | 100.0 |
| | | 14 | 100.0 | 100.0 |
| | | 28 | 100.0 | 100.0 |
| SEC | Aggregate% | 0 | 0.4 | 0.4 |
| | | 14 | 0.4 | 0.4 |
| | | 28 | 0.9 | 1.0 |
| | Main peak% | 0 | 99.0 | 99.0 |
| | | 14 | 99.2 | 99.2 |
| | | 28 | 98.8 | 98.7 |
| | Fragment% | 0 | 0.6 | 0.6 |
| | | 14 | 0.3 | 0.3 |
| | | 28 | 0.3 | 0.3 |
| | **Change in aggregate (%)** | | **0.5** | **0.6** |
| | **Change in main peak (%)** | | **-0.2** | **-0.3** |
| | **Change in fragment (%)** | | **-0.3** | **-0.3** |
| CEX | Acidic peak% | 0 | 17.3 | 17.1 |
| | | 14 | 18.1 | 18.5 |
| | | 28 | 18.7 | 18.7 |
| | Main peak% | 0 | 51.6 | 51.8 |
| | | 14 | 51.4 | 49.5 |
| | | 28 | 50.8 | 50.9 |
| | Basic peak% | 0 | 31.2 | 31.0 |
| | | 14 | 30.4 | 32.0 |
| | | 28 | 30.5 | 30.4 |
| | **Change in acidic peak (%)** | | **1.4** | **1.6** |
| | **Change in main peak (%)** | | **-0.8** | **-0.9** |
| | **Change in basic peak (%)** | | **-0.7** | **-0.6** |
| CE-SDS | Intact IgG% | 0 | 96.8 | 96.8 |
| | | 14 | 96.4 | 96.5 |
| | | 28 | 97.7 | 97.7 |
| | Intact heavy chain% | 0 | 94.1 | 93.9 |
| | | 14 | 93.8 | 93.7 |
| | | 28 | 95.3 | 95.3 |
| | **Change in intact IgG (%)** | | **0.9** | **0.9** |
| | **Change in intact heavy chain (%)** | | **1.2** | **1.4** |
| FlowCam particles/mL | 1~2 µm | 0 | 26 | 26 |
| | | 7 | 32 | 43 |
| | | 14 | 108 | 96 |
| | | 28 | 885 | 566 |
| | 2~5 µm | 0 | 43 | 60 |
| | | 7 | 113 | 77 |
| | | 14 | 224 | 340 |
| | | 28 | 1770 | 707 |
| | 5~10 µm | 0 | 17 | 26 |
| | | 7 | 24 | 0 |
| | | 14 | 17 | 96 |
| | | 28 | 159 | 33 |
| | 10~25 µm | 0 | 17 | 43 |
| | | 7 | 16 | 9 |
| | | 14 | 8 | 26 |
| | | 28 | 42 | 8 |
| | 25 µm+ | 0 | 0 | 0 |
| | | 7 | 0 | 0 |
| | | 14 | 0 | 8 |
| | | 28 | 0 | 0 |
| Note: [1] Appearance description is divided into two aspects: opalescence and particles. Degree of opalescence: * denotes clarity, ** denotes slight opalescence, *** denotes heavy opalescence, and **** denotes opacification; degree of particles: - denotes no particles visible to the naked eye, and + denotes the presence of particles visible to the naked eye. | | | | |

As shown in Table 18 and FIGs. 7f-7j, after 10 freeze-thaw cycles under a condition of -20 °C to room temperature, there were no significant changes in the basic physicochemical properties (appearance, protein concentration, pH, osmotic pressure), subvisible particle (SVP) level, average protein particle size, and purity (SEC, CEX, and CE-SDS) of the proteins in each formulation; the freeze-thaw study results indicated that the freeze-thaw stability of the anti-TIGIT antibody protein in each formulation was good. Therefore, based on the studies on protein aggregation, degradation, and charge heterogeneity, the polysorbate 80 concentrations of 0.03% and 0.05% can both maintain the stability of the anti-TIGIT antibody protein, with 0.03% being the preferred concentration for polysorbate 80.

### Example 8. Buffer Concentration and Buffer System Optimization

Example 3 in the present application preliminarily determined that the suitable buffer concentration range is 5-15 mM, and the lower the buffer concentration, the better the protein stability. However, the applicant found that during the sucrose and polysorbate concentration studies conducted in the examples described above, when using an acetate buffer system with a concentration of 5 mM, the formulation would experience pH drift after being subjected to concentration and buffer exchange in a centrifuge tube, such as shown in Table 23, where the pH would increase by 0.1-0.2. Since pH drift increases the difficulty of controlling the concentration and buffer exchange process, this example investigated buffers and buffer concentrations that prevent pH drift.

### 8.1 Buffer concentration optimization

In this example, as shown in Table 22, an acetate buffer system, a pH of 5.1, buffer concentrations of 10 mmol/L, 15 mmol/L, 20 mmol/L, and 25 mmol/L, a protein concentration of 20.0 mg/mL, 3.42% sucrose, and 0.02% polysorbate 80 (PS80) were selected for the formulations. Four candidate formulations were designed using the single-factor approach.

**Table 22. Formulation information for screening study on the buffer system of anti-TIGIT antibody formulations**

| **Formulation No.** | **Buffer concentration mM** | **Buffer system** | **Protein concentration, mg/mL** | **pH** | **Sucrose content, %** | **Polysorbate 80 content, %** |
|---|---|---|---|---|---|---|
| F1-10 mM acetate | 10 | Acetate buffer | 20.0 | 5.1 | 3.42 | 0.02 |
| F2-15 mM acetate | 15 | | | | | |
| F3-20 mM acetate | 20 | | | | | |
| F4-25 mM acetate | 25 | | | | | |

As shown in Table 23, the formulations containing acetate buffer (with buffer concentrations of 10 mM, 15 mM, 20 mM, and 25 mM) experienced an increase in pH by 0.1-0.2 after being subjected to concentration and buffer exchange in a centrifuge tube, and the lower the buffer concentration, the greater the pH increase, indicating that increasing the concentration of acetate buffer does not reduce the pH drift phenomenon after the concentration and buffer exchange for formulations. On day 0, there were no significant differences in appearance, protein concentration, molecular variant (SEC, CE-SDS), and charge variant (CEX) among the various formulations. The initial aggregation temperatures (Tagg) were ranked as: F1-10 mM acetate (62.9 °C) > F2-15 mM acetate (62.1 °C) > F3-20 mM acetate (61.4 °C) > F4-25 mM acetate (60.8 °C); the initial average protein particle sizes were ranked as: F1-10 mM acetate (5.2 nm) < F2-15 mM acetate (6.1 nm) < F3-20 mM acetate (6.7 nm) < F4-25 mM acetate (7.3 nm).

After the formulations described above were kept at 40 °C for 28 days, all the formulations had no particles visible to the naked eye and maintained a good appearance; there were also no significant differences in subvisible particle (SVP) levels. The average protein particle sizes were ranked as: F1-10 mM acetate (5.7 nm) < F2-15 mM acetate (6.4 nm) < F3-20 mM acetate (7.0 nm) < F4-25 mM acetate (7.6 nm).

As shown in FIGs. 8a and 8b, the CE-SDS and SEC fragment results indicated that there were no significant differences in purity among the various formulations. As shown in FIG. 8c, the CEX results showed that decreases in main peak and basic peak contents occurred along with an increase in acidic peak content, and there were no significant differences in the acidic peak increases among the formulations. Moreover, in view of the buffer concentration study in Example 3, the buffer concentration for formulations comprising the anti-TIGIT antibody can be 10 mM-15 mM, preferably 10 mM or 15 mM. Furthermore, based on the results of protein aggregates, fragments, average particle size, etc., the acetate buffer can be used as the formulation buffer.

**Table 23. Summary of key data from screening study on the buffer concentration of anti-TIGIT antibody formulations (40 °C)**

| **Detection item** | | **Investigation time** | **F1** | **F2** | **F3** | **F4** |
|---|---|---|---|---|---|---|
| | | | **10 mM acetate buffer** | **15 mM acetate buffer** | **20 mM acetate buffer** | **25 mM acetate buffer** |
| Basic physicochemical detection | T_{agg}°C | 0 days | 62.9 | 62.1 | 61.4 | 60.8 |
| | Appearance^{[1]} | 0 days | **/- | **/- | **/- | **/- |
| | | 7 days | **/- | **/- | **/- | **/- |
| | | 14 days | **/- | **/- | **/- | **/- |
| | | 28 days | **/- | **/- | **/- | **/- |
| | Protein concentration | 0 days | 19.6 | 19.6 | 19.5 | 20.7 |
| | | 28 days | 19.1 | 19.7 | 19.2 | 20.5 |
| | pH | 0 days | 5.3 | 5.3 | 5.2 | 5.2 |
| | | 28 days | 5.3 | 5.2 | 5.1 | 5.1 |
| | pH of buffer | | 5.1 | 5.1 | 5.1 | 5.1 |
| | pH drift | | 0.2 | 0.2 | 0.1 | 0.1 |
| DLS | Average particle size nm | 0 days | 5.2 | 6.1 | 6.7 | 7.3 |
| | | 7 days | 5.5 | 6.2 | 6.8 | 7.4 |
| | | 14 days | 5.5 | 6.3 | 6.8 | 7.3 |
| | | 28 days | 5.7 | 6.4 | 7.0 | 7.6 |
| | Pk1 | 0 days | 100.0 | 100.0 | 100.0 | 97.7 |
| | | 7 days | 100.0 | 100.0 | 100.0 | 100.0 |
| | | 14 days | 100.0 | 100.0 | 100.0 | 100.0 |
| | | 28 days | 100.0 | 100.0 | 100.0 | 100.0 |
| SEC | Aggregate% | 0 days | 0.4 | 0.4 | 0.4 | 0.6 |
| | | 7 days | 0.3 | 0.4 | 0.4 | 0.5 |
| | | 14 days | 0.4 | 0.4 | 0.5 | 0.7 |
| | | 28 days | 0.4 | 0.4 | 0.4 | 0.7 |
| | Main peak% | 0 days | 99.3 | 99.3 | 99.2 | 99.1 |
| | | 7 days | 99.2 | 99.3 | 99.3 | 99.1 |
| | | 14 days | 98.7 | 98.7 | 98.6 | 98.4 |
| | | 28 days | 98.5 | 98.5 | 98.5 | 98.3 |
| | Fragment% | 0 days | 0.3 | 0.3 | 0.4 | 0.4 |
| | | 7 days | 0.5 | 0.4 | 0.4 | 0.4 |
| | | 14 days | 0.9 | 0.9 | 0.9 | 1.0 |
| | | 28 days | 1.1 | 1.0 | 1.1 | 1.1 |
| | **Change in aggregate (%)** | | **0.0** | **0.0** | **0.0** | **0.1** |
| | **Change in main peak (%)** | | **-0.8** | **-0.8** | **-0.7** | **-0.8** |
| | **Change in fragment (%)** | | **0.8** | **0.7** | **0.7** | **0.7** |
| CEX | Acidic peak% | 0 days | 18.5 | 18.2 | 18.5 | 18.6 |
| | | 7 days | 21.4 | 21.7 | 21.8 | 21.9 |
| | | 14 days | 24.8 | 24.9 | 25.2 | 25.3 |
| | | 28 days | 31.4 | 31.8 | 32.0 | 32.1 |
| | Main peak% | 0 days | 49.5 | 50.1 | 49.8 | 49.9 |
| | | 7 days | 47.5 | 47.2 | 47.3 | 47.3 |
| | | 14 days | 41.8 | 41.4 | 41.3 | 41.4 |
| | | 28 days | 41.9 | 41.5 | 41.2 | 41.0 |
| | Basic peak% | 0 days | 32.0 | 31.7 | 31.7 | 31.5 |
| | | 7 days | 31.1 | 31.1 | 30.9 | 30.8 |
| | | 14 days | 33.4 | 33.7 | 33.5 | 33.3 |
| | | 28 days | 26.7 | 26.8 | 26.8 | 27.0 |
| | **Change in acidic peak (%)** | | **12.9** | **13.6** | **13.5** | **13.5** |
| | **Change in main peak (%)** | | **-7.6** | **-8.6** | **-8.6** | **-8.9** |
| | **Change in basic peak (%)** | | **-5.3** | **-4.9** | **-4.9** | **-4.5** |
| CE-SDS | Intact IgG% | 0 days | 97.8 | 97.9 | 98.2 | 98.0 |
| | | 7 days | 97.7 | 97.5 | 97.5 | 97.5 |
| | | 14 days | 95.5 | 95.3 | 95.4 | 95.1 |
| | | 28 days | 95.0 | 94.9 | 94.8 | 94.7 |
| | Intact heavy chain% | 0 days | 95.7 | 95.6 | 95.6 | 95.5 |
| | | 7 days | 94.7 | 94.8 | 94.7 | 94.8 |
| | | 14 days | 93.9 | 94.0 | 93.5 | 94.0 |
| | | 28 days | 90.6 | 91.0 | 90.5 | 90.6 |
| | **Change in intact IgG (%)** | | **-2.8** | **-3.0** | **-3.4** | **-3.3** |
| | **Change in intact heavy chain (%)** | | **-5.1** | **-4.6** | **-5.1** | **-4.9** |
| FlowCam particles/mL | 1~2 µm | 0 days | 34 | 2223 | 224 | 289 |
| | | 7 days | 347 | 124 | 527 | 52 |
| | | 14 days | 69 | 924 | 1790 | 126 |
| | | 28 days | 84 | 68 | 120 | 503 |
| | 2~5 µm | 0 days | 68 | 3599 | 332 | 632 |
| | | 7 days | 828 | 142 | 1236 | 156 |
| | | 14 days | 164 | 2552 | 2890 | 143 |
| | | 28 days | 193 | 272 | 215 | 1198 |
| | 5~10 µm | 0 days | 17 | 596 | 42 | 114 |
| | | 7 days | 223 | 27 | 268 | 26 |
| | | 14 days | 17 | 84 | 264 | 8 |
| | | 28 days | 92 | 51 | 40 | 243 |
| | 10~25 µm | 0 days | 8 | 218 | 25 | 35 |
| | | 7 days | 89 | 0 | 164 | 43 |
| | | 14 days | 0 | 53 | 51 | 8 |
| | | 28 days | 17 | 17 | 8 | 84 |
| | 25 µm+ | 0 days | 17 | 17 | 0 | 0 |
| | | 7 days | 0 | 0 | 0 | 0 |
| | | 14 days | 0 | 0 | 0 | 0 |
| | | 28 days | 8 | 0 | 0 | 0 |
| Note: [1] Appearance description is divided into two aspects: opalescence and particles. Degree of opalescence: * denotes clarity, ** denotes slight opalescence, *** denotes heavy opalescence, and **** denotes opacification; degree of particles: - denotes no particles visible to the naked eye, and + denotes the presence of particles visible to the naked eye. | | | | | | |

### 8.2 Buffer system optimization

Given that formulations with acetate buffer exhibit pH drift after concentration and buffer exchange, and that pH drift increases the difficulty of controlling the concentration and buffer exchange process, the applicant optimized the buffer system to obtain a buffer that prevents pH drift.

In this example, an acetate buffer or histidine buffer, a pH of 5.1, a protein concentration of 20.0 mg/mL, 3.42% sucrose, 0.02% polysorbate 80 (PS80), and a buffer concentration of 10 mM were selected for the formulations. Two candidate formulations were designed using the single-factor approach (as shown in Table 24).

**Table 24. Formulation information for screening study on the buffer system of the anti-TIGIT antibody**

| **Formulation No.** | **Buffer system** | **Protein concentration, mg/mL** | **pH** | **Buffer concentration mM** | **Sucrose content, %** | **Polysorbate 80 content, %** |
|---|---|---|---|---|---|---|
| F1 | Acetate buffer | 20.0 | 5.1 | 10 | 3.42 | 0.02 |
| F2 | Histidine buffer | | | | | |

As shown in Table 25, the formulation with acetate buffer (with a buffer concentration of 10 mM) experienced an increase in pH by 0.2 after concentration and buffer exchange; the formulation with histidine buffer (with a buffer concentration of 10 mM) did not experience pH change after concentration and buffer exchange. In the high-temperature accelerated testing, on day 0, there were no significant differences in appearance, protein concentration, initial average protein particle size, molecular variant (SEC, CE-SDS), and charge variant (CEX) among the various formulations. The initial protein aggregation temperatures (Tagg) were ranked as: F1-acetate buffer (62.9 °C) > F2-histidine buffer (60.9 °C). Although the Tagg temperature of the acetate buffer formulation was slightly higher than that of the histidine buffer formulation, there were no significant differences. It can be considered that 10 mM histidine buffer is conducive to maintaining the pH stability of the anti-TIGIT antibody formulation and can also maintain the stability of the anti-TIGIT antibody protein.

As shown in Table 25, after 28 days of standing at 40 °C, there were no significant differences in appearance, subvisible particle level, average protein particle size, and SEC aggregate among the formulations. Moreover, as shown in Table 25 and FIG. 8d, the CE-SDS and SEC fragment results indicated that there were no significant differences among the different formulations. Therefore, based on the analysis results of protein aggregates and degradation, the buffer can be an acetate buffer or a histidine buffer with a concentration of 10 mM.

As shown in Table 25 and FIG. 8e, the CEX results showed that decreases in main peak and basic peak contents occurred along with an increase in acidic peak content, and the acidic peak increases for the formulations were ranked as: F1-10 mM acetate buffer (12.9%) > F2-10 mM histidine buffer (10.9%). Compared to the formulation with acetate buffer, the formulation with histidine buffer had less acidic peak formation. Therefore, based on the analysis results of charge heterogeneity, the histidine buffer is superior to the acetate buffer in reducing protein charge heterogeneity.

In conclusion, both the acetate buffer and histidine buffer can maintain the stability of the anti-TIGIT antibody and inhibit the production of acidic peaks. Therefore, the buffer choice for formulations comprising the anti-TIGIT antibody can be either an acetate buffer or a histidine buffer, with a concentration of 10 mM-15 mM. Furthermore, since the pH of the histidine buffer formulation does not drift, the buffer is preferably a histidine buffer.

**Table 25. Summary of key data from screening study on the buffer system of anti-TIGIT antibody formulations (40 °C)**

| **Detection item** | | **Investigation time** | **F1** | **F2** |
|---|---|---|---|---|
| | | | **10 mM acetate buffer** | **10 mM histidine buffer** |
| Basic physicochemical detection | T_{agg}°C | 0 days | 62.9 | 60.9 |
| | Appearance^{[1]} | 0 days | **/- | **/- |
| | | 7 days | **/- | **/- |
| | | 14 days | **/- | **/- |
| | | 28 days | **/- | **/- |
| | Protein concentration | 0 days | 19.6 | 19.4 |
| | | 28 days | 19.1 | 19.4 |
| | pH | 0 days | 5.3 | 5.1 |
| | | 28 days | 5.3 | 5.1 |
| | pH of buffer | | 5.1 | 5.1 |
| | pH drift | | 0.2 | 0.0 |
| DLS | Average particle size nm | 0 days | 5.2 | 5.7 |
| | | 7 days | 5.5 | 5.8 |
| | | 14 days | 5.5 | 5.8 |
| | | 28 days | 5.7 | 6.0 |
| | Pk1 | 0 days | 100.0 | 100.0 |
| | | 7 days | 100.0 | 100.0 |
| | | 14 days | 100.0 | 100.0 |
| | | 28 days | 100.0 | 100.0 |
| SEC | Aggregate% | 0 days | 0.4 | 0.5 |
| | | 7 days | 0.3 | 0.2 |
| | | 14 days | 0.4 | 0.2 |
| | | 28 days | 0.4 | 0.3 |
| | Main peak% | 0 days | 99.3 | 99.4 |
| | | 7 days | 99.2 | 99.5 |
| | | 14 days | 98.7 | 99.4 |
| | | 28 days | 98.5 | 99.1 |
| | Fragment% | 0 days | 0.3 | 0.2 |
| | | 7 days | 0.5 | 0.2 |
| | | 14 days | 0.9 | 0.4 |
| | | 28 days | 1.1 | 0.7 |
| | **Change in aggregate (%)** | | **0.0** | **-0.2** |
| | **Change in main peak (%)** | | **-0.8** | **-0.3** |
| | **Change in fragment (%)** | | **0.8** | **0.5** |
| CEX | Acidic peak% | 0 days | 18.5 | 18.5 |
| | | 7 days | 21.4 | 20.9 |
| | | 14 days | 24.8 | 23.7 |
| | | 28 days | 31.4 | 29.4 |
| | Main peak% | 0 days | 49.5 | 49.9 |
| | | 7 days | 47.5 | 47.4 |
| | | 14 days | 41.8 | 41.8 |
| | | 28 days | 41.9 | 41.9 |
| | Basic peak% | 0 days | 32.0 | 31.6 |
| | | 7 days | 31.1 | 31.7 |
| | | 14 days | 33.4 | 34.5 |
| | | 28 days | 26.7 | 28.7 |
| | **Change in acidic peak (%)** | | **12.9** | **10.9** |
| | **Change in main peak (%)** | | **-7.6** | **-8.0** |
| | **Change in basic peak (%)** | | **-5.3** | **-2.9** |
| CE-SDS | Intact IgG% | 0 days | 97.8 | 98.1 |
| | | 7 days | 97.7 | 97.4 |
| | | 14 days | 95.5 | 95.7 |
| | | 28 days | 95.0 | 94.4 |
| | Intact heavy chain% | 0 days | 95.7 | 95.5 |
| | | 7 days | 94.7 | 95.0 |
| | | 14 days | 93.9 | 93.9 |
| | | 28 days | 90.6 | 90.6 |
| | **Change in intact IgG (%)** | | **-2.8** | **-3.7** |
| | **Change in intact heavy chain (%)** | | **-5.1** | **-4.9** |
| FlowCam particles/mL | 1~2 µm | 0 days | 34 | 367 |
| | | 7 days | 347 | 137 |
| | | 14 days | 69 | 312 |
| | | 28 days | 84 | 186 |
| | 2~5 µm | 0 days | 68 | 953 |
| | | 7 days | 828 | 361 |
| | | 14 days | 164 | 443 |
| | | 28 days | 193 | 642 |
| | 5~10 µm | 0 days | 17 | 166 |
| | | 7 days | 223 | 52 |
| | | 14 days | 17 | 78 |
| | | 28 days | 92 | 110 |
| | 10~25 µm | 0 days | 8 | 79 |
| | | 7 days | 89 | 26 |
| | | 14 days | 0 | 26 |
| | | 28 days | 17 | 34 |
| | 25 µm+ | 0 days | 17 | 17 |
| | | 7 days | 0 | 0 |
| | | 14 days | 0 | 0 |
| | | 28 days | 8 | 0 |
| Note: [1] Appearance description is divided into two aspects: opalescence and particles. Degree of opalescence: * denotes clarity, ** denotes slight opalescence, *** denotes heavy opalescence, and **** denotes opacification; degree of particles: - denotes no particles visible to the naked eye, and + denotes the presence of particles visible to the naked eye. | | | | |

### 8.3 pH value optimization

In view of the pH and the effects of pH drift factors determined in the examples described above, it is necessary to further investigate the stability of the anti-TIGIT antibody protein in formulations within a pH range of 5.1-5.7. This example selected the preferred formulations from the examples described above: an anti-TIGIT antibody protein with a concentration of 20 mg/mL, a histidine buffer with a concentration of 10 mM, sucrose with a concentration of 20 mg/mL (2%), and polysorbate 80 with a concentration of 0.3 mg/mL (0.03%). Three candidate formulations were designed using the single-factor approach (as shown in Table 26), and the stability of the anti-TIGIT antibody protein at different pH values was evaluated through the high-temperature (40 °C) accelerated testing. Sampling and testing were carried out according to the experimental design in Table 27.

**Table 26. Formulation information for confirmation study on the pH range of anti-TIGIT antibody formulations**

| **Formulation No.** | **pH** | **Buffer system** | **Protein concentration, mg/mL** | **Buffer concentration mM** | **Sucrose content (%)** | **Polysorbate 80 content (%)** |
|---|---|---|---|---|---|---|
| F1-pH 5.1 | 5.1 | Histidine buffer | 20.0 | 10 | 2 | 0.03 |
| F2-pH 5.4 | 5.4 | | | | | |
| F3-pH 5.7 | 5.7 | | | | | |

**Table 27. Investigation conditions and detection methods for confirmation study on the pH range of anti-TIGIT antibody formulations**

| **Study content** | **Investigation condition** | **Detection point/detection method** | | |
|---|---|---|---|---|
| | | **0 days** | **14 days** | **28 days** |
| High temperature | 40 °C | X, Y | X | X, Y |
| X: appearance, protein concentration (A₂₈₀), SEC, DLS, FlowCam, icIEF, NR&R CE-SDS | | | | |
| Y: pH | | | | |

As shown in Table 28, on day 0, the appearance of the formulations with different pH values was a colorless and slightly opalescent liquid with no particles visible to the naked eye, and these formulations had small protein particle size, low levels of SEC aggregates, fragments, and acidic peak content, and high levels of intact antibody and heavy chain contents.

After 28 days of standing at 40 °C, the appearance of the formulations with different pH values was still a colorless and slightly opalescent liquid with no particles visible to the naked eye; the FlowCam results showed no significant changes in subvisible particles. As shown in Table 28 and FIGs. 8f and 8g, compared to day 0, the average protein particle size increased by only 0.1-0.2 nm, and the particle size increase was not significant; Pk1 remained at 100%, and SEC aggregates increased by 0.3%-0.5%, indicating a small aggregate increase. Therefore, based on the analysis results of average protein particle size and aggregates, the anti-TIGIT antibody protein showed good stability in various formulations at pH 5.1, 5.4, and 5.7, and the anti-TIGIT antibody protein showed the best stability when the pH value was 5.4.

As shown in Table 28 and FIGs. 8h and 8i, the CE-SDS and icIEF results showed that the anti-TIGIT antibody protein degradation and the increase in acidic peaks were less in formulations at pH 5.1, 5.4, and 5.7, and the anti-TIGIT antibody protein degradation and the increase in acidic peaks were minimal in the formulation at pH 5.4. Therefore, based on the analysis results of protein degradation and charge heterogeneity, the anti-TIGIT antibody protein showed good stability in various formulations at pH 5.1, 5.4, and 5.7, and the anti-TIGIT antibody protein showed the best stability when the pH value was 5.4.

In conclusion, the anti-TIGIT antibody protein in the formulations described above was more stable within the pH range of 5.1-5.7, with optimal protein stability achieved at pH 5.4.

**Table 28. Summary of key data from confirmation study on the pH range for the anti-TIGIT antibody (40 °C)**

| **Detection item** | | **Investigation time** | **F1** | **F2** | **F3** |
|---|---|---|---|---|---|
| | | | **pH 5.1** | **pH 5.4** | **pH 5.7** |
| Basic physicochemical detection | Appearance^{[1]} | 0 days | **/- | **/- | **/- |
| | | 14 days | **/- | **/- | **/- |
| | | 28 days | **/- | **/- | **/- |
| | Protein concentration | 0 days | 20.1 | 19.8 | 19.8 |
| | | 28 days | 18.5 | 18.6 | 18.5 |
| | pH | 0 days | 5.1 | 5.4 | 5.7 |
| | | 28 days | 5.2 | 5.5 | 5.8 |
| DLS | Average particle size nm | 0 days | 5.8 | 5.7 | 5.7 |
| | | 14 days | 5.9 | 5.8 | 5.9 |
| | | 28 days | 6.0 | 5.8 | 5.9 |
| | Pk1 | 0 days | 100.0 | 100.0 | 100.0 |
| | | 14 days | 100.0 | 100.0 | 100.0 |
| | | 28 days | 100.0 | 100.0 | 100.0 |
| SEC | Aggregate% | 0 days | 0.4 | 0.3 | 0.4 |
| | | 14 days | 0.6 | 0.4 | 0.5 |
| | | 28 days | 0.9 | 0.6 | 0.8 |
| | Main peak% | 0 days | 99.5 | 99.5 | 99.4 |
| | | 14 days | 99.0 | 99.2 | 99.0 |
| | | 28 days | 98.5 | 98.8 | 98.5 |
| | Fragment% | 0 days | 0.2 | 0.2 | 0.2 |
| | | 14 days | 0.4 | 0.4 | 0.5 |
| | | 28 days | 0.6 | 0.7 | 0.7 |
| | **Change in aggregate (%)** | | **0.5** | **0.3** | **0.4** |
| | **Change in main peak (%)** | | **-1.0** | **-0.7** | **-0.9** |
| | **Change in fragment (%)** | | **0.3** | **0.5** | **0.5** |
| icIEF | Acidic peak% | 0 days | 16.6 | 16.5 | 16.4 |
| | | 14 days | 24.9 | 24.4 | 23.6 |
| | | 28 days | 33.9 | 32.3 | 33.0 |
| | Main peak% | 0 days | 58.0 | 58.5 | 58.1 |
| | | 14 days | 52.3 | 52.7 | 53.2 |
| | | 28 days | 46.8 | 47.8 | 47.4 |
| | Basic peak% | 0 days | 25.4 | 24.9 | 25.5 |
| | | 14 days | 22.8 | 22.8 | 23.2 |
| | | 28 days | 19.3 | 19.8 | 19.6 |
| | **Change in acidic peak (%)** | | **17.3** | **15.8** | **16.6** |
| | **Change in main peak (%)** | | **-11.2** | **-10.7** | **10.7** |
| | **Change in basic peak (%)** | | **-6.1** | **-5.1** | **-5.9** |
| CE-SDS | Intact IgG% | 0 days | 98.2 | 98.4 | 98.4 |
| | | 14 days | 97.9 | 98.0 | 97.9 |
| | | 28 days | 96.0 | 96.3 | 96.1 |
| | Intact heavy chain% | 0 days | 96.6 | 96.5 | 96.0 |
| | | 14 days | 95.8 | 95.9 | 95.4 |
| | | 28 days | 94.2 | 94.5 | 94.2 |
| | **Change in intact IgG (%)** | | **-2.2** | **-2.1** | **-2.3** |
| | **Change in intact heavy chain (%)** | | **-2.4** | **-2.0** | **-1.8** |
| FlowCam particles/mL | 1~2 µm | 0 days | 410 | 188 | 335 |
| | | 14 days | 791 | 270 | 671 |
| | | 28 days | 57 | 122 | 24 |
| | 2~5 µm | 0 days | 683 | 417 | 671 |
| | | 14 days | 947 | 343 | 1216 |
| | | 28 days | 98 | 179 | 170 |
| | 5~10 µm | 0 days | 162 | 49 | 303 |
| | | 14 days | 201 | 123 | 165 |
| | | 28 days | 33 | 98 | 97 |
| | 10~25 µm | 0 days | 34 | 74 | 65 |
| | | 14 days | 78 | 25 | 215 |
| | | 28 days | 16 | 81 | 40 |
| | 25 µm+ | 0 days | 0 | 8 | 0 |
| | | 14 days | 11 | 0 | 0 |
| | | 28 days | 0 | 0 | 0 |
| Note: [1] Appearance description is divided into two aspects: opalescence and particles. Degree of opalescence: * denotes clarity, ** denotes slight opalescence, *** denotes heavy opalescence, and **** denotes opacification; degree of particles: - denotes no particles visible to the naked eye, and + denotes the presence of particles visible to the naked eye. | | | | | |

Discussion of results: In view of the pH value screening study, stabilizer and concentration study, buffer and concentration screening study, and surfactant screening study in the examples described above, a formulation capable of maintaining the stability of the anti-TIGIT antibody was obtained. In the study on stabilizers, sucrose, sorbitol, and proline can all maintain the stability of the anti-TIGIT antibody. Considering the crystallization risk of sorbitol during the long-term frozen storage of the stock solution, the cost of proline as an auxiliary material for pharmaceutical formulations is high. In view of the studies in the examples described above, sucrose as a stabilizer can better maintain the stability of the anti-TIGIT antibody in formulations. Therefore, sucrose is the preferred stabilizer for the formulation of the present disclosure. Additionally, the anti-TIGIT antibody has good freeze-thaw stability within the sucrose concentration range of 2%-6%, and the initial average particle size of the anti-TIGIT antibody is optimal at a sucrose concentration of 2%. In the buffer screening, both the acetate buffer and histidine buffer (histidine-histidine hydrochloride buffer) can maintain the stability of the anti-TIGIT antibody. However, in view of the stability of pH value, the histidine buffer is the preferred buffer. In the buffer concentration screening study, the selectable buffer concentration range is 5-15 mM, and the preferred concentration is 10 mM. In view of the buffer and its concentration, a pH value within the range of 5.1-5.7 can maintain the stability of the anti-TIGIT antibody protein, and the anti-TIGIT antibody is most stable at a pH value of 5.4. In the surfactant screening study, both polysorbate 20 and polysorbate 80 can maintain the stability of the anti-TIGIT antibody protein in the pharmaceutical formulation, and polysorbate 80, compared to polysorbate 20, exhibits to a slower growth of anti-TIGIT antibody protein particle size and a monodisperse particle size distribution, which is more conducive to the stability of the anti-TIGIT antibody. Additionally, the selectable concentrations of polysorbate 20 or 80 range from 0.03%-0.05%, with the viable options being 0.03% and 0.05%, and the preferred option being 0.03%. Based on the studies in the examples described above, the preferred scheme for the anti-TIGIT antibody formulation is shown in Table 29, and the preferred scheme for the anti-TIGIT antibody formulation is shown in Table 30.

**Table 29. The preferred anti-TIGIT antibody formulation**

| **Buffer** | **Protein concentration mg/mL** | **pH value** | **Stabilizer and content** | **Surfactant and content** |
|---|---|---|---|---|
| 5-15 mM histidine buffer or acetate buffer | 15~25.0 | 5.1~5.7 | 2%-6% sucrose | 0.01%-0.06% polysorbate 80 or polysorbate 20 |

**Table 30. The most preferred anti-TIGIT antibody formulation**

| **Buffer** | **Protein concentration mg/mL** | **pH value** | **Stabilizer and content** | **Surfactant and content** |
|---|---|---|---|---|
| 10 mM histidine buffer | 20 | 5.4 | 2% sucrose | 0.03% polysorbate 80 |

For the preferred formulation of the anti-TIGIT antibody protein as shown in Table 30, the high-temperature (40 °C) accelerated testing was conducted to verify the stability of the anti-TIGIT antibody protein in this formulation. The study conditions and detection methods are shown in Table 31.

**Table 31. Investigation conditions and detection methods for anti-TIGIT antibody formulation study**

| **Investigation condition** | **Detection point/detection method** | | |
|---|---|---|---|
| | **0 days** | **14 days** | **28 days** |
| 40 °C | X, Y | X | X, Y |
| X: appearance, protein concentration (A₂₈₀), SEC, DLS, FlowCam, icIEF, NR CE-SDS | | | |
| Y: pH | | | |

On day 0, the appearance of the formulation was a colorless and slightly opalescent liquid with no particles visible to the naked eye, and the formulation had low levels of aggregates and fragments (both 0.1%), a small average protein particle size (5.4 nm), a low acidic peak content (17.6%), and a high level of intact IgG (98.2%).

After 28 days of standing at 40 °C, as shown in Table 32 and FIGs. 9a-9b, the appearance of the formulation was still a colorless and slightly opalescent liquid with no particles visible to the naked eye; the FlowCam results showed no significant increases in subvisible particles. Compared to day 0, the average protein particle size increased by only 0.2 nm, and the particle size increase was not significant; particle size distribution still remained monomodal; SEC aggregates increased by 0.2%, indicating a small aggregate increase. Therefore, based on the aggregation analysis results, the stability of the anti-TIGIT antibody in this formulation was good; as shown in Table 32 and FIGs. 9c-9d, after 28 days of standing at 40 °C, SEC fragments increased by 0.3%; the CE-SDS results showed a decrease of 1.4% in intact IgG; the icIEF results showed an increase of 12.5% in acidic peaks, with main peak and basic peaks decreasing by 9.2% and 3.4%, respectively.

Overall, in the formulation described above, the degradation of the anti-TIGIT antibody protein and the increase in acidic peaks were less, indicating good stability. In summary, the analysis results of protein degradation and charge heterogeneity indicated that the stability of the anti-TIGIT antibody protein in the formulation described above was good.

**Table 32. Summary of key data from anti-TIGIT antibody formulation study (40 °C)**

| **Detection item** | | **Investigation time** | **Detection results** |
|---|---|---|---|
| Basic physicochemical detection | Appearance^{[1]} | 0 days | **/- |
| | | 14 days | **/- |
| | | 28 days | **/- |
| | Protein concentration | 0 days | 19.2 |
| | | 28 days | 20.5 |
| | pH | 0 days | 5.4 |
| | | 28 days | 5.4 |
| DLS | Average particle size nm | 0 days | 5.4 |
| | | 14 days | 5.5 |
| | | 28 days | 5.6 |
| | Pk1 | 0 days | 100.0 |
| | | 14 days | 100.0 |
| | | 28 days | 100.0 |
| SEC | Aggregate% | 0 days | 0.1 |
| | | 14 days | 0.4 |
| | | 28 days | 0.3 |
| | Main peak% | 0 days | 99.9 |
| | | 14 days | 99.4 |
| | | 28 days | 99.3 |
| | Fragment% | 0 days | 0.1 |
| | | 14 days | 0.3 |
| | | 28 days | 0.4 |
| | Change in aggregate (%) | | 0.2 |
| | Change in main peak (%) | | -0.6 |
| | Change in fragment (%) | | 0.3 |
| icIEF | Acidic peak% | 0 days | 17.6 |
| | | 14 days | 23.8 |
| | | 28 days | 30.1 |
| | Main peak% | 0 days | 55.8 |
| | | 14 days | 51.8 |
| | | 28 days | 46.6 |
| | Basic peak% | 0 days | 26.7 |
| | | 14 days | 24.4 |
| | | 28 days | 23.3 |
| | Change in acidic peak (%) | | 12.5 |
| | Change in main peak (%) | | -9.2 |
| | Change in basic peak (%) | | -3.4 |
| CE-SDS | Intact IgG% | 0 days | 98.2 |
| | | 14 days | 97.7 |
| | | 28 days | 96.8 |
| | Change in intact IgG (%) | | -1.4 |
| FlowCam particles/mL | 1~2 µm | 0 days | 264 |
| | | 14 days | 116 |
| | | 28 days | 158 |
| | 2~5 µm | 0 days | 691 |
| | | 14 days | 447 |
| | | 28 days | 564 |
| | 5~10 µm | 0 days | 119 |
| | | 14 days | 41 |
| | | 28 days | 49 |
| | 10~25 µm | 0 days | 17 |
| | | 14 days | 33 |
| | | 28 days | 40 |
| | 25 µm+ | 0 days | 8 |
| | | 14 days | 0 |
| | | 28 days | 20 |
| Note: [1] Appearance description is divided into two aspects: opalescence and particles. Degree of opalescence: * denotes clarity, ** denotes slight opalescence, *** denotes heavy opalescence, and **** denotes opacification; degree of particles: - denotes no particles visible to the naked eye, and + denotes the presence of particles visible to the naked eye. | | | |

The anti-TIGIT antibody formulation described above was stored at -40 °C for at least 24 h, and then subjected to 3 repeated freeze-thaw cycles and long-term frozen storage according to the repeated freeze-thaw conditions shown in Table 33 and the long-term frozen storage conditions shown in Table 34, respectively, for appearance, protein concentration, pH, average protein particle size, Flowcam, SEC, icIEF, and CE-SDS detection.

**Table 33. Repeated freeze-thaw conditions**

| **Investigation condition** | **Repeated freeze-thaw condition** | | |
|---|---|---|---|
| **Freeze-thaw, -40 °C to RT** | **T0** | **1 cycle** | **3 cycles** |
| Sample volume 1 mL | X | | |
| Detection items: X = appearance, UV_280, pH, SEC, DLS, FlowCam, CE-SDS, icIEF | | | |

**Table 34. Long-term frozen storage conditions**

| **Investigation condition** | **Long-term frozen storage condition** | | | | |
|---|---|---|---|---|---|
| **-40 °C** | **0 months** | **1.5 months** | **3 months** | **6.5 months** | **18 months** |
| Sample volume 1 mL | X | | | | |
| Detection items: X = appearance, UV_280, pH, SEC, DLS, FlowCam, CE-SDS, icIEF | | | | | |

Referring to Table 35, after 3 repeated freeze-thaw cycles of the anti-TIGIT antibody formulation, the appearance of the formulation remained a colorless and slightly opalescent liquid with no foreign matter or particles visible to the naked eye, and the formulation had a stable protein concentration and pH value, no significant increase in average protein particle size, no significant increase in SEC aggregates, and no significant increase in SEC fragments; the icIEF results showed no significant changes in acidic peaks, main peak, and basic peaks; the CE-SDS results showed no significant changes in NR-MP%; no significant change trend was observed in the number of Flowcam particles.

**Table 35. Detection data for the repeated freeze-thaw cycles of the anti-TIGIT antibody formulation**

| **Detection item** | **Number of cycles** | **Detection results** | |
|---|---|---|---|
| Appearance | 0 | Colorless, slightly opalescent, free of obvious visible foreign matter | |
| | 1 | Colorless, slightly opalescent, free of obvious visible foreign matter | |
| | 3 | Colorless, slightly opalescent, free of obvious visible foreign matter | |
| Protein Conc. (mg/mL) | 0 | 19.2 | |
| | 1 | 19.1 | |
| | 3 | 19.3 | |
| pH | 0 | 5.4 | |
| | 1 | 5.4 | |
| | 3 | 5.4 | |
| DLS | Average particle size, nm | 0 | 5.4 |
| | | 1 | 5.4 |
| | | 3 | 5.4 |
| SEC | Aggregate% | 0 | 0.1 |
| | | 1 | 0.1 |
| | | 3 | 0.1 |
| | Monomer% | 0 | 99.9 |
| | | 1 | 99.8 |
| | | 3 | 99.8 |
| | Fragment% | 0 | 0.1 |
| | | 1 | 0.1 |
| | | 3 | 0.1 |
| icIEF | Acidic peak% | 0 | 17.6 |
| | | 1 | 17.6 |
| | | 3 | 17.0 |
| | Main peak% | 0 | 55.8 |
| | | 1 | 55.6 |
| | | 3 | 56.2 |
| | Basic peak% | 0 | 26.7 |
| | | 1 | 26.8 |
| | | 3 | 26.8 |
| CE-SDS | NR-MP% | 0 | 98.2 |
| | | 1 | 98.1 |
| | | 3 | 98.4 |

Referring to Table 36, after 18 months of long-term frozen storage of the anti-TIGIT antibody formulation, the appearance of the formulation remained a colorless and slightly opalescent liquid with no foreign matter or particles visible to the naked eye, and the formulation had a stable protein concentration and pH value, no significant increase in average protein particle size, no significant increase in SEC aggregates, and no significant increase in SEC fragments; the icIEF results showed no significant changes in acidic peaks, main peak, and basic peaks; the CE-SDS results showed no significant changes in NR-MP%, NGHC, and R-MP; no significant change trend was observed in the number of Flowcam particles.

Therefore, the pharmaceutical formulation comprising the anti-TIGIT antibody provided by the present disclosure can maintain the stability of the anti-TIGIT antibody during long-term frozen storage and repeated freeze-thaw cycles.

**Table 36. Detection data for the long-term frozen storage of the anti-TIGIT antibody formulation**

| **Detection item** | | **Month** | **Detection results** |
|---|---|---|---|
| Appearance | | 0 | Colorless, slightly opalescent, free of obvious visible foreign matter |
| | | 1.5 | Colorless, slightly opalescent, free of obvious visible foreign matter |
| | | 3 | Colorless, slightly opalescent, free of obvious visible foreign matter |
| | | 6.5 | Colorless, slightly opalescent, free of obvious visible foreign matter |
| | | 18 | Colorless, slightly opalescent, free of obvious visible foreign matter |
| Protein concentration mg/mL | | 0 | 19.2 |
| | | 1.5 | 20.1 |
| | | 3 | 20.0 |
| | | 6.5 | 19.8 |
| | | 18 | 20.0 |
| SEC | Aggregate% | 0 | 0.1 |
| | | 1.5 | 0.2 |
| | | 3 | 0.3 |
| | | 6.5 | 0.2 |
| | | 18 | 0.2 |
| | Monomer% | 0 | 99.9 |
| | | 1.5 | 99.7 |
| | | 3 | 99.6 |
| | | 6.5 | 99.7 |
| | | 18 | 99.7 |
| | Fragment% | 0 | 0.1 |
| | | 1.5 | 0.1 |
| | | 3 | 0.1 |
| | | 6.5 | 0.1 |
| | | 18 | 0.1 |
| DLS | Average particle size, nm | 0 | 5.4 |
| | | 1.5 | 5.5 |
| | | 3 | 5.4 |
| | | 6.5 | 5.9 |
| | | 18 | 5.5 |
| icIEF | Acidic peak% | 0 | 17.6 |
| | | 1.5 | 18.8 |
| | | 3 | 17.1 |
| | | 6.5 | 16.4 |
| | | 18 | 15.3 |
| | Main peak% | 0 | 55.8 |
| | | 1.5 | 55.1 |
| | | 3 | 56.0 |
| | | 6.5 | 56.7 |
| | | 18 | 57.5 |
| | Basic peak% | 0 | 26.7 |
| | | 1.5 | 26.1 |
| | | 3 | 26.9 |
| | | 6.5 | 26.9 |
| | | 18 | 27.0 |
| CE-SDS | NR-MP% | 0 | 98.2 |
| | | 1.5 | 98.6 |
| | | 3 | 98.9 |
| | | 6.5 | 98.9 |
| | | 18 | 98.4 |
| | NGHC | 0 | N/A |
| | | 1.5 | 0.7 |
| | | 3 | 0.7 |
| | | 6.5 | 0.8 |
| | R-MP | 0 | N/A |
| | | 1.5 | 96.9 |
| | | 3 | 96.5 |
| | | 6.5 | 97.0 |

It should be understood that various modifications or changes may be made by those skilled in the art after reading the aforementioned content of the present disclosure, and these equivalent forms also fall within the scope defined by the appended claims of the present application.

## Claims

1. A pharmaceutical formulation, comprising:
(i) an anti-TIGIT antibody, wherein the anti-TIGIT antibody is a single-domain antibody, and a heavy chain variable region of the anti-TIGIT antibody comprises or consists of an amino acid sequence of SEQ ID NO: 1;
(ii) 5-15 mM histidine buffer;
(iii) 2% (w/v)-6% (w/v) sucrose; and
(iv) 0.01% (w/v)-0.05% (w/v) polysorbate 80;
wherein the pharmaceutical formulation has a pH value of 5.1 to 5.7.

2. The pharmaceutical formulation according to claim 1, wherein the anti-TIGIT antibody has a concentration of 15-25 mg/mL.

3. The pharmaceutical formulation according to claim 1 or 2, wherein the anti-TIGIT antibody has a concentration of 20 mg/mL.

4. The pharmaceutical formulation according to any one of claims 1-3, wherein a full-length heavy chain of the anti-TIGIT antibody comprises or consists of an amino acid sequence of SEQ ID NO: 2.

5. The pharmaceutical formulation according to any one of claims 1-4, comprising:
the anti-TIGIT antibody at 20 mg/mL;
10 mM histidine buffer;
2% (w/v) sucrose; and
0.03% (w/v) polysorbate 80;
wherein the pharmaceutical formulation has a pH value of 5.1 to 5.7.

6. The pharmaceutical formulation according to any one of claims 1-5, wherein the pharmaceutical formulation preferably has a pH value of 5.4.

7. The pharmaceutical formulation according to any one of claims 1-6 for use in treating a cancer in a subject.

8. The pharmaceutical formulation for use according to claim 7, wherein the cancer is a solid tumor or a hematological tumor.

9. The pharmaceutical formulation for use according to claim 7 or 8, wherein the cancer is selected from the group consisting of mesothelioma, lung cancer, pancreatic cancer, ovarian cancer, breast cancer, colon cancer, pleural tumor, glioblastoma, esophageal cancer, synovial sarcoma, thymic carcinoma, cervical cancer, endometrial cancer, gastric tumor, melanoma, cholangiocarcinoma, head and neck cancer, lymphoma, hematological cancer, and a combination thereof.

10. The pharmaceutical formulation for use according to any one of claims 7-9, wherein the pharmaceutical formulation is administered in combination with an additional agent.

11. The pharmaceutical formulation for use according to claim 10, wherein the additional agent is an anti-PD-1 antibody and/or an anti-PD-L1 antibody.

12. The pharmaceutical formulation for use according to claim 10 or 11, wherein the additional agent is an anti-PD-1 antibody.

13. Use of the pharmaceutical formulation according to any one of claims 1-6 in manufacrure of a medicament for treating a cancer in a subject.

14. The use according to claim 13, wherein the cancer is a solid tumor or a hematological tumor.

15. The use according to claim 13 or 14, wherein the cancer is selected from the group consisting of mesothelioma, lung cancer, pancreatic cancer, ovarian cancer, breast cancer, colon cancer, pleural tumor, glioblastoma, esophageal cancer, synovial sarcoma, thymic carcinoma, cervical cancer, endometrial cancer, gastric tumor, melanoma, cholangiocarcinoma, head and neck cancer, lymphoma, hematological cancer, and a combination thereof.

16. The use according to any one of claims 13-15, wherein the pharmaceutical formulation is administered in combination with an additional agent.

17. The use according to claim 16, wherein the additional agent is an anti-PD-1 antibody and/or an anti-PD-L1 antibody.

18. The use according to claim 16 or 17, wherein the additional agent is an anti-PD-1 antibody.

19. A method for reducing tumor burden in a patient and/or treating a tumor in a patient, comprising administering to the patient an effective dose of the pharmaceutical formulation according to any one of claims 1-6.

20. The method according to claim 19, wherein the tumor is a solid tumor or a hematological tumor.

21. The method according to claim 19 or 20, wherein the tumor is selected from the group consisting of mesothelioma, lung cancer, pancreatic cancer, ovarian cancer, breast cancer, colon cancer, pleural tumor, glioblastoma, esophageal cancer, synovial sarcoma, thymic carcinoma, cervical cancer, endometrial cancer, gastric tumor, cholangiocarcinoma, head and neck cancer, lymphoma, hematological cancer, and a combination thereof.

22. The method according to any one of claims 19-21, wherein the pharmaceutical formulation is administered in combination with an additional agent.

23. The method according to claim 22, wherein the additional agent is an anti-PD-1 antibody and/or an anti-PD-L1 antibody.

24. The method according to claim 22 or 23, wherein the additional agent is an anti-PD-1 antibody.

25. A drug delivery device, comprising the pharmaceutical formulation according to any one of claims 1-6.
